# FASCICULE DE BREVET EUROPEEN

(11) **EP 0 623 620 B1**
(45) Date de publication et mention de la délivrance du brevet: **09.09.1998**
(21) Numéro de dépôt: 94400881.2
(22) Date de dépôt: 25.04.1994
(51) Int. Cl.: C07D 487/04, A61K 31/495, C07D 471/14, C07D 487/14

(54) **Dérivés de pyrrolopyrazines à activité 5-HT3**
5-HT3 Pyrrolopyrazinderivate
5-HT3 Pyrrolopyrazine derivatives

(30) Priorité: 30.04.1993 FR 9305109
(43) Date de publication de la demande: 09.11.1994
(73) Titulaire: ADIR ET COMPAGNIE, 92415 Courbevoie Cédex (FR)
(72) Inventeur: Rault, Sylvain, F-14370 Moult (FR); Lancelot, Jean-Charles, F-14400 Le Bourg (FR); Prunier, Hervé, F-14000 Caen (FR); Robba, Max, F-75004 Paris (FR); Delagrange, Philippe, F-92130 Issy les Moulineaux (FR); Renard, Pierre, F-78000 Versailles (FR); Adam, Gérard, F-78600 Le Mesnil le Roi (FR)

(56) Documents cités:
- JOURNAL OF MEDICINAL CHEMISTRY vol. 33 , 190 , WASHINGTON US pages 2087 - 2093 J.E. MANCOR ET AL. '3--(1,2,5,6-Tetrahydro pyrid-4-yl)pyrrolo[3,2-b]pyrid-5-0ne: A potent and selective serotonin (5-HT 1B] agonist and rotationally restricted phenolic analogue of 5-methooxy-3-(1,2,5,6 -tetrahydropyrid-4-yl)indole'
- CHEMICAL ABSTRACTS, vol. 77, no. 19, 1972, Columbus, Ohio, US; abstract no. 126567y, K. NAGARAJAN ET AL. 'Condensed heterocycles. Pyrrolo[1,2-a]quinoxaline derivatives' page 396 ;

## Description

La présente invention concerne de nouveaux composés pyrrolopyraziniques, leurs procédés de préparation et les compositions pharmaceutiques qui les contiennent.

La demanderesse a présentement découvert de nouveaux composés pyrrolopyraziniques qui montrent une très forte affinité pour les récepteurs 5-HT₃ sélective vis-à-vis des autres récepteurs sérotoninergiques.

Le potentiel et l'intérêt thérapeutique d'agents pharmacologiques agissant sur les récepteurs 5-HT₃ a été mentionné de nombreuses fois, notamment dans la revue "5-HT₃ receptors" (Kilpatrick G.J., Medicinal Research Reviews, 1990, 10 (4), pp 441-475).

On connait, dans la littérature, des pyrroloquinoxalines (Campiani G. et al, Synthetic Communications, 1991, 21 (15-16), pp 1567-1576, Nagarajan K. et al Indian J. Chem. 1972, 10, pp 344-350). Parmi ces pyrroloquinoxalines, on connait surtout la 4-(4-méthylpipérazinyl)-7-(trifluorométhyl)pyrrolo[1,2-a]quinoxaline, qui est décrite exclusivement comme un agoniste des récepteurs sérotoninergiques 5-HT_{1B} (Neale R.F. et al, Eur. J. of Pharmacology, 1987, 136, pp 1-9 et Macor J. E. et al J. Med. Chem. 1990, 33, pp 2087-2093).

Plus particulièrement, la présente invention concerne les composés de formule (I) : 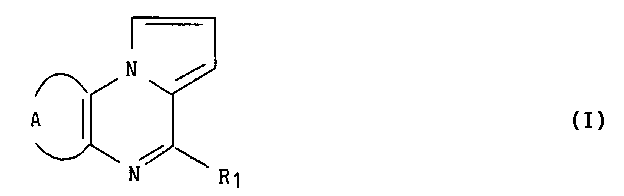 dans laquelle :
R₁ représente un groupement de formule : 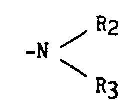 dans laquelle R₂ et R₃ forment avec l'atome d'azote qui les porte un groupement choisi parmi :
- pipérazine,
- pipérazine substituée,
- pipéridine,
- pipéridine substituée,
- pyrrolidine,
- pyrrolidine substituée,
- morpholine,
- morpholine substituée par un ou plusieurs radicaux alkyles,
- tétrahydropyridine,
- thiomorpholine,
- azaspirane de 5 à 12 chaînons,
- azaspirane de 5 à 12 chaînons substitué par un ou plusieurs radicaux alkyles ou groupements oxo,
- azacycloalkyle mono ou bicyclique de 7 à 12 chaînons incluant éventuellement dans son squelette de 1 à 2 hétéroatomes supplémentaires choisis parmi oxygène, soufre, et azote,
- azacycloalkyle mono ou bicyclique de 7 à 12 chaînons, substitué par un ou plusieurs radicaux alkyles ou groupements oxo, incluant éventuellement dans son squelette de 1 à 2 hétéroatomes supplémentaires choisis parmi oxygène, soufre, et azote,
- un groupement -NH-(CH₂)ₖ-NH₂ dans lequel k représente un nombre entier égal à 2, 3, ou 4,
- et un groupement -NH-(CH₂)ₖ-NH₂ substitué dans lequel k est tel que défini précédemment,
et A forme avec les 2 atomes de carbone auxquels il est lié un cycle choisi parmi benzo, pyrido, pyrazino, et pyrimidino ; A étant non substitué ou substitué par un ou plusieurs radicaux choisis parmi :
- alkyle,
- hydroxy,
- alkoxy,
- acyle,
- alkoxycarbonyle,
- halogène,
- trifluorométhyle,
- -(CH₂)ₘ-phényle et -O-(CH₂)ₘ-phényle dans lequel le noyau phényle est lui-même non substitué ou substitué par un ou plusieurs radicaux choisis parmi halogène, alkyle, alkoxy, hydroxy, et trifluorométhyle ; et m représente O ou un nombre entier de 1 à 4,
- -(CH₂)ₘ-pipérazine dans lequel le groupement pipérazine est lui-même substitué ou non substitué et m est tel que défini précédemment.
étant entendu que si A forme avec les 2 atomes de carbone auxquels il est lié un cycle benzo non substitué ou substitué par un trifluorométhyle ou un fluor, alors R₂ et R₃ ne peuvent pas former avec l'atome d'azote qui les porte une pipérazine non substituée ou substituée en position 4 par un radical méthyle ou (méthyl)phényle, et que si A forme avec les 2 atomes de carbone auxquels il est lié un cycle benzo non substitué, alors R₂ et R₃ ne peuvent pas former avec l'atome d'azote qui les porte une morpholine ou un groupement diméthylaminopropylamino,
étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux oxo, radicaux R₄, ou radicaux 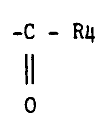 avec R₄ étant choisi parmi :
- alkyle,
- alkoxy,
- alcényle non substitué ou substitué par un radical phényle lui-même non substitué ou substitué par un ou plusieurs radicaux choisis parmi halogène, alkyle, alkoxy, hydroxy et trifluorométhyle,
- -(CH₂)ₙ-R₅ ou 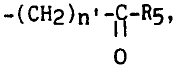 où n représente 0 ou un nombre entier de 1 à 5, n' représente un nombre entier de 1 à 5, et où R₅ représente un radical choisi parmi phényle, benzhydryle, thiényle, pyrrolyle, pyrrolidinyle, furyle, pyrimidinyle, pyridyle, méthylènedioxyphényle, éthylènedioxyphényle, naphtyle, quinoléinyle, isoquinoléinyle, cycloalkyle, et dicycloalkylméthyl ; le terme "cycloalkyle" représentant un groupement mono- ou bi-cyclique de 3 à 12 chaînons,
   ces radicaux R₅ pouvant eux-mêmes être substitués par un ou plusieurs radicaux choisis parmi halogène, trifluorométhyle, carboxyle, hydroxyle, alkyle, alkoxy,
- et -(CH₂)_{n'} -R₆ où n' est tel que défini précédemment et R₆ représente un groupement choisi parmi carboxyle, alkoxycarbonyle, amino, alkylamino, dialkylamino, -SO₂NR₇R₈, et -CONR₇R₈ dans lesquels R₇ et R₈ représentent chacun indépendamment l'un de l'autre un atome d'hydrogène ou un alkyle,
leurs isomères optiques,
et leurs sels d'addition à un acide ou à une base pharmaceutiquement acceptable,
étant entendu que, sauf précisions contraires,
- les termes "alkyle", "alkoxy", et "acyle" représentent des groupements linéaires ou ramifiés possédant de 1 à 6 atomes de carbone,
- et le terme "alcényle" représente un groupement insaturé, linéaire ou ramifié, possédant de 2 à 6 atomes de carbone.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

L'invention concerne par exemple :
- les composés de formule (I) dans lesquels A forme avec les 2 atomes de carbone auxquels il est lié un cycle benzo,
- les composés de formule (I) dans lesquels A forme avec les 2 atomes de carbone auxquels il est lié un cycle pyrido,
- et les composés de formule (I) dans lesquels R₂ et R₃ forment avec l'atome d'azote qui les porte une pipérazine substituée en position 4 par
un radical benzyle lui-même non substitué ou substitué, leurs isomères optiques,
et leurs sels d'addition à un acide ou à une base pharmaceutiquement acceptable.

La présente invention s'étend au procédé de préparation des composés de formule (I) caractérisé en ce que :
· soit on porte au reflux, en présence de phosgène, un composé de formule (II), 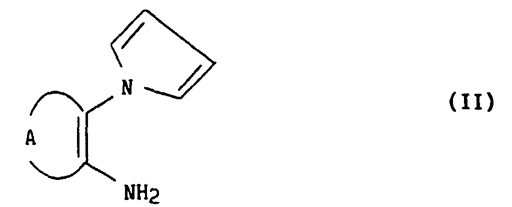 dans laquelle A est tel que défini dans la formule (I),
· soit on chauffe, en présence ou en absence de solvant, un composé de formule (II/1), 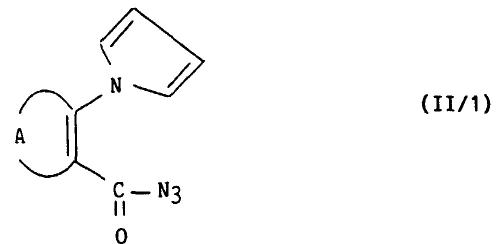 dans laquelle A est tel que défini dans la formule (I),
pour obtenir un composé de formule (I/a) : 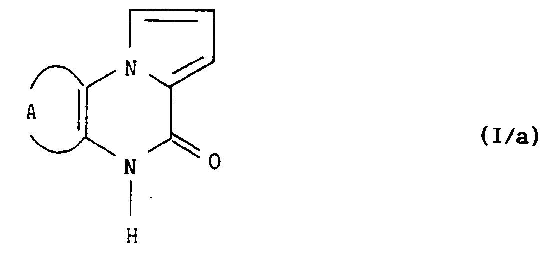 dans laquelle A est tel que défini précédemment,
composé de formule (I/a) qui est soumis à un agent d'halogénation pour obtenir un composé de formule (I/b) : 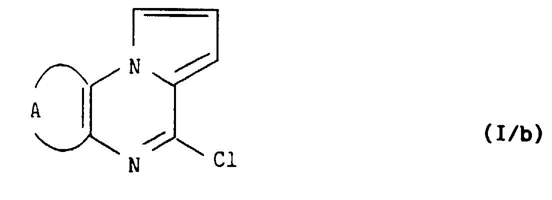 dans laquelle A est tel que défini précédemment,
qui est ensuite mis en réaction avec une amine de formule (III) : 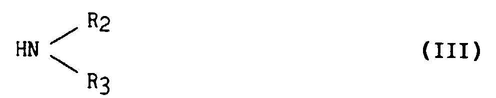 dans laquelle R₂ et R₃ sont tels que définis dans la formule (I),
pour obtenir un composé de formule (I) : 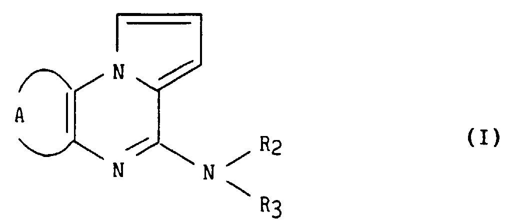 dans laquelle A, R₂, et R₃ sont tels que définis précédemment,
les composés de formule (I) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou par une base pharmaceutiquement acceptable.

L'invention s'étend également au procédé de préparation des composés de formule (I),
caractérisé en ce que :
on fait réagir un composé de formule (II') : 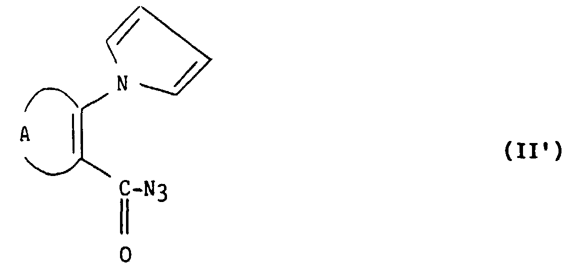 dans laquelle A est tel que défini dans la formule (I),
avec une amine de formule (III) : 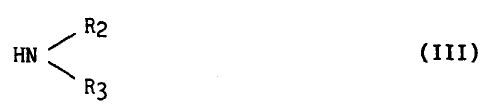 dans laquelle R₂ et R₃ sont tels que définis dans la formule (I) pour obtenir un composé de formule (V) : 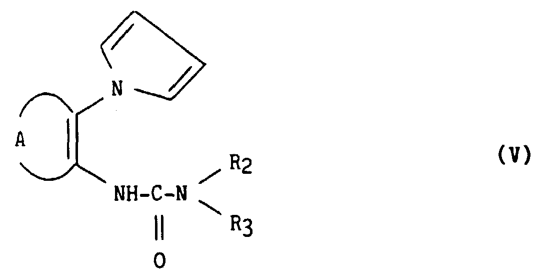 dans laquelle A, R₂ et R₃ sont tels que définis précédemment,
qui est ensuite cyclisé sous l'action d'un agent d'halogénation et soumis à un traitement alcalin pour donner un composé de formule (I) : 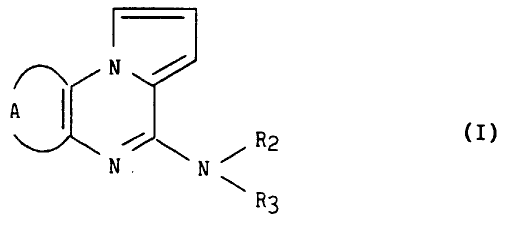 dans laquelle A, R₂, et R₃ sont tels que définis précédemment,
les composés de formule (I) pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou par une base pharmaceutiquement acceptable.

Il est également possible d'accéder aux composés de formule (I) dans lesquels le substituant de A avec A tel que défini dans la formule (I) et le groupement R₁ avec R₁ tel que défini dans la formule (I) sont identiques par greffage simultané du substituant de A et de R₁.

Les matières premières utilisées dans les procédés précédemment décrits sont :
- soit commerciales,
- soit aisément accessibles à l'homme de métier selon des procédés décrits dans la littérature.

Les composés de formule (I) possèdent des propriétés pharmacologiques très intéressantes.

La demanderesse a découvert que les composés de l'invention possédaient, de façon sélective, une haute affinité pour les récepteurs sérotoninergiques 5-HT₃.

En outre, leur puissante affinité pour les récepteurs 5-HT₃ a été démontrée aussi bien in vitro (mesure de l'accumulation de ¹⁴C-guanidinium dans les cellules NG 108-15, exemple B de la présente demande) qu'in vivo (mesure du réflexe de BEZOLD-JARISCH, exemple C de la présente demande).

Les composés de l'invention sont donc susceptibles d'être utilisés dans la prévention et le traitement de l'anxiété, de la dépression, du stress, des psychoses, de la schizophrénie, des troubles du système nerveux central, de la migraine, des troubles de la mémoire, des troubles du comportement alimentaire, de l'alcoolisme, de la douleur, ainsi que dans la prévention et le traitement du vomissement et des désordres de la vidange gastrique.

L'invention s'étend aussi aux compositions pharmaceutiques renfermant comme principe actif au moins un des composés de formule (I) ou un de ses sels d'addition avec un acide ou avec un base pharmaceutiquement acceptable en combinaison avec un ou plusieurs excipients ou véhicules pharmaceutiquement acceptables.

Parmi les compositions selon l'invention, on pourra citer, à titre d'exemples et de façon non limitative, celles qui conviennent pour l'administration orale, parentérale, oculaire, per ou trans-cutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, et les gels.

Les préparations ainsi obtenues se présentent généralement sous forme dosée et peuvent contenir selon les affections traitées, l'âge, et le sexe du malade de 0,01 à 100 mg de principe actif en prises de une à trois fois par jour, de préférence de 0,01 à 5 mg de principe actif, particulièrement de 0,1 à 5 mg, par exemple 1 mg.

Les exemples qui suivent illustrent l'invention et ne la limitent en aucune façon.

### EXEMPLE 1 : 4-(4-BENZYLPIPERAZINO)-PYRROLO[1,2-a]QUINOXALINE

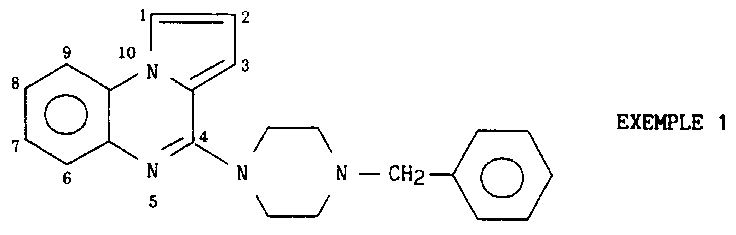

**Stade A : 4,5-dihydro-4-oxo-pyrrolo[1,2-a]quinoxaline** 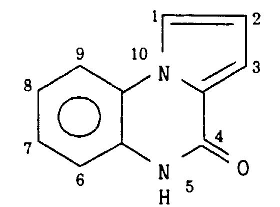
   On porte au reflux pendant 1h 30 min, dans 200 cm³ de toluène, 15g (0,095 mole) de 2-(pyrrol-1-yl)-aniline en présence de 50 cm³ de phosgène à 20 % en solution toluènique. Après refroidissement, le précipité formé est essoré, lavé à l'éther éthylique, séché et recristallisé dans l'acétonitrile (poudre blanche sublimée à 270°C).
   Rendement : 80 %
   Caractéristiques spectrales :
   Infra rouge νcm⁻¹(KBr) : 1640(C=0), bandes principales à 1360, 1080, 725 et 640.
   Spectre de RMN (DMSO d₆) :
   δH pyrroliques : 8,13 ; 7,00 : 6,63 ; δH C₆H₄ : 7,96 ; 7,23
**Stade A' :**
   **2ème procédé**
   On ajoute goutte à goutte à 0°C à une solution de 20g (0,106 mole) d'acide 2-(pyrrol-1-yl)benzoïque dans un mélange (50-50) d'acétone, d'acétonitrile et 14,69 cm³ (d = 0,73) de triéthylamine. Après 30 min d'agitation, on ajoutte goutte à goutte 10,13 cm³ (d = 1,135) de chloroformiate d'éthyle de façon à ce que la température reste comprise entre 0 et + 5°C, puis, après 30 min d'agitation, 6,89 g (0,106 mole) d'azoture de sodium en solution dans 30 cm³ d'eau. Le mélange réactionnel est agité à 0°C pendant 2h puis versé sur 600 cm³ d'eau. La solution est extraite par 150 cm³ d'éther, la phase organique est décantée, séchée et évaporée sous pression réduite. L'azoture formé se transforme spontanément de manière dangereuse en 4,5-dihydro-4-oxo-pyrrolo[1,2-a]quinoxaline par réaménagement. Sublimation à 270°C.
**Stade B : 4-chloro-pyrrolo[1,2-a]quinoxaline** 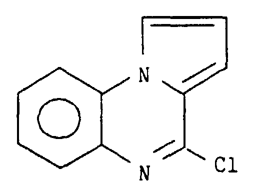 10g (0,0543 mole) de 4,5-dihydro-4-oxo-pyrrolo[1,2-a]quinoxaline sont portés au reflux dans 180 cm³ d'oxychlorure de phosphore et 10 cm³ de pyridine pendant 5 h. Après avoir été à sec, le résidu est repris par 300 cm³ d'eau, agité puis neutralisé par une solution d'ammoniaque aqueuse. Le précipité est essoré, lavé à l'eau, séché et recristallisé dans l'acétate d'éthyle.
   Point de fusion : 172°C
   Rendement : 63 %
   Caractéristiques spectrales
   Infrarouge νcm⁻¹ (KBr) : bandes principales à : 1600, 1410, 750 et 720.
   Spectre de RMN (DMSOd₆) : δH pyrroliques : 8,53 ; 7,00 ; δH6, H7, H8, H9 : 8,26 ; 7,80 ; 7,50
**Stade C** : **4-(4-benzylpipérazino)-pyrrolo[1,2-a]quinoxaline**
   On porte à 140°C pendant 2h 30 min dans 30 cm³ de diméthylformamide un mélange de 2g (0,0098 mole) de 4-chloro-pyrrolo[1,2-a]quinoxaline, 1,72g de 1-benzylpipérazine et de 1,04 g (0,0098 mole) de carbonate de sodium. Après refroidissement, la solution est versée dans 100 cm³ d'eau sous agitation puis extraite par 150 cm³ d'éther éthylique. La phase éthérée est lavée avec 100 cm³ d'eau, décantée, séchée puis concentrée sous pression réduite. L'huile résiduelle jaune foncée est mise en solution dans 30 cm³ d'alcool isopropylique puis additionnée par 3 cm³ d'acide chlorhydrique concentré. Après 30 min d'agitation, le précipité est essoré, lavé avec 10 cm³ d'alcool isopropylique puis avec 20 cm³ d'éther éthylique anhydre. Solvant de recristallisation : acétonitrile (poudre blanche).
   Point de fusion (trichlorhydrate) : 182°C
   Rendement : 51 %
   Caractéristiques spectrales :
   Infrarouge : νcm⁻¹ (KBr) 2550, 2650, 2880 NH⁺, bandes principales à : 1500, 1580, 1410, 940 et 680.
   Spectre de RMN (DMSOd₆) : δH C₆H₅ : 7,47 ; δH C₆H₅ : 8,17 ; 7,63 ; δH pyrroliques : 8,61 ; 7,47 et 6,98 ; δH CH₂ 4,67 ; 4,43 ; 3,43 ; δNH⁺ : 4,70

### EXEMPLE 2 : 4-[4-(4-FLUOROBENZYL)PIPERAZINO]-PYRROLO[1,2-a]QUINOXALINE

En procédant comme dans l'exemple 1 mais en remplaçant au stade C la 1-benzylpipérazine par la 1-(4-fluorobenzyl)pipérazine, on obtient le composé du titre.
Solvant de recristallisation : acétonitrile (poudre blanche).
Point de fusion (trichlorhydrate) : 182°C
Rendement : 49 %
Caractéristiques spectrales :
Infrarouge : νcm⁻¹ (KBr) 2560, 2660, 2880 NH⁺, bandes principales à : 1580, 1420, 1220 et 745
Spectre de RMN (DMSOd₆) :δH pyrroliques : 8,60 ; 7,36 et 7,00 ; δH C₆H₄-F : 7,73 et 7,36, δH C₆H₄-F : 8,20 et 7,36, δH CH₂ : 4,46 ; 3,43 ; δHNH⁺ : 4,46

### EXEMPLE 3 A 10 :

En procédant comme dans les exemples 1 ou 2 mais en utilisant au stade A la 2-(pyrrol-1-yl)-aniline convenablement substituée et au stade C la 1-benzylpipérazine ou la 1-(4-fluorobenzyl)pipérazine, on obtient les composés des exemples suivants :

### EXEMPLE 3 : 4-(4-BENZYLPIPERAZINO)-7-CHLORO-PYRROLO[1,2-a]QUINOXALINE

**Stade A : 7-chloro-4,5-dihydro-4-oxo-pyrrolo[1,2-a]quinoxaline**
   Solvant de recristallisation : acétonitrile
   Point de fusion : > 270°C
   Rendement : 76 %
**Stade B : 4,7-dichloro-pyrrolo[1,2-a]quinoxaline**
   Solvant de recristallisation : acétonitrile
   Point de fusion : 204°C
   Rendement : 59 %
**Stade C : 4-(4-benzylpipérazino)-7-chloro-pyrrolo[1,2-a]quinoxaline**
   Solvant de recristallisation : acétonitrile
   Point de fusion (trichlorhydrate) : 168°C
   Rendement : 46 %
   Infrarouge : νcm⁻¹ (KBr) 2600, 2700 NH⁺, bandes principales à : 1600, 1510, 960 et 760.
   Spectre de RMN (DMSOd₆) : δH pyrroliques 8,53 ; 7,33 ; 6,93 ; δH₆, H₈, H₉ : 8,13 ; 7,60 ; δH C₆H₅ : 7,33 ; δH NH⁺ 4,83 ; δH CH₂ : 4,43 ; 4,06 ; 3,36

### EXEMPLE 4 : 7-CHLORO-4-[4-(4-FLUOROBENZYL)PIPERAZINO]-PYRROLO[1,2-a] QUINOXALINE

Solvant de recristallisation : acétonitrile
Point de fusion (trichlorhydrate) : 212°C
Rendement : 49 %
Caractéristiques spectrales :
Infrarouge : νcm⁻¹ (KBr) 2620, 2720 (NH⁺), bandes principales à : 1600, 1515, 1230, 810 et 770.
Spectre de RMN (DMSOd₆) : δH pyrroliques 8,56 ; 7,33 ; 6,93 ; δH₆, H₈, H₉ : 8,13 ; 7,33 ; δH C₆H₄-F : 7,76 ; 7,33 ; δH NH⁺: 5,00 ; δH CH₂: 4,40 ; 4,00 ; 3,33

### EXEMPLE 5 : 4-(4-BENZYLPIPERAZINO)-8-CHLORO-PYRROLO[1,2-a]QUINOXALINE

**Stade A : 8-chloro-4,5-dihydro-4-oxo-pyrrolo[1,2-a]quinoxaline**
   Solvant de recristallisation : acétonitrile
   Point de fusion : > 270°C
   Rendement : 64 %
**Stade B : 4,8-dichloro-pyrrolo[1,2-a]quinoxaline**
   Solvant de recristallisation : acétonitrile
   Point de fusion : 188°C
   Rendement : 57 %
**Stade C : 4-(4-benzylpipérazino)-8-chloro-pyrrolo[1,2-a]quinoxaline**
   Solvant de recristallisation : acétonitrile
   Point de fusion (trichlorhydrate) : 210°C
   Rendement : 37 %
   Caractéristiques spectrales :
   Infrarouge :
   νcm⁻¹ (KBr) 2600, 2700 (NH⁺), bandes principales à : 1595, 1430, 1390, 1120, 750 et 705.
   Spectre de RMN (DMSOd₆) : δH pyrroliques 8,53 ; 6,93 ; δH₆, H₇, H₉ : 8,26 ; 8,10 ; 7,66 ; δH C₆H₅ : 7,36 ; δH NH⁺ : 4,80 ; δH CH₂ : 4,40 ; 4,03 ; 3,36

### EXEMPLE 6 : 8-CHLORO-4-[4-(4-FLUOROBENZYL)PIPERAZINO]-PYRROLO[1,2-a]QUINOXALINE

### EXEMPLE 7 : 4-[4-(4-FLUOROBENZYL)PIPERAZINO]-7-METHYL-PYRROLO[1,2-a]QUINOXALINE

**Stade A : 4,5-dihydro-7-méthyl-4-oxo-pyrrolo[1,2-a]quinoxaline**
   Solvant de recristallisation : acétonitrile
   Sublimation à 240°C
   Rendement : 40 %
**Stade B : 4-chloro-7-méthyl-pyrrolo[1,2-a]quinoxaline**
   Solvant de recristallisation : acétate d'éthyle
   Point de fusion : 158°C
   Rendement : 53 %
**Stade C : 4-[4-(4-fluorobenzyl)pipérazino]-7-méthyl-pyrrolo[1,2-a]quinoxaline**
   Solvant de recristallisation : acétonitrile
   Point de fusion (trichlorhydrate) : 206°C
   Caractéristiques spectrales :
   Infrarouge : νcm⁻¹ (KBr) 2340, 2540, 2600 NH⁺, bandes principales à : 1605, 1520, 1240, 960 et 770.
   Spectre de RMN (DMSOd₆) : δH pyrroliques 8,55 ; 7,20 ; 7,00 ; δH₆, H₈, H₉ : 8,13 ; 7,96 ; 7,33 ; δH C₆H₄-F : 7,73 ; 7,33 ; δH NH⁺ : 4,66 ; δH CH₂ : 4,40 ; 4,10 ; 3,40 ; δH CH₃ : 2,40

### EXEMPLE 8 : 4-(4-BENZYLPIPERAZINO)-7-METHYL-PYRROLO[1,2-a]QUINOXALINE

### EXEMPLE 9 : 4-[4-(4-FLUOROBENZYL)PIPERAZINO]-7-METHOXY-PYRROLO[1,2-a]QUINOXALINE

**Stade A : 4,5-dihydro-7-méthoxy-4-oxo-pyrrolo[1,2-a]quinoxaline**
   Solvant de recristallisation : acétonitrile
   Sublimation à 250°C
**Stade B : 4-chloro-7-méthoxy-pyrrolo[1,2-a]quinoxaline**
   Solvant de recristallisation : acétate d'éthyle
   Point de fusion : 132°C
   Rendement : 34 %
**Stade C : 4-[4-(4-fluorobenzyl)pipérazino]-7-méthoxy-pyrrolo[1,2-a]quinoxaline**
   Solvant de recristallisation : acétonitrile
   Point de fusion : 220°C
   Caractéristiques spectrales :
   Infrarouge : νcm⁻¹ (KBr) 2600, 2700 NH⁺, bandes principales à : 1600, 1510, 1235, 810 et 770.
   Spectre de RMN (DMSOd₆) : δH pyrroliques 8,56 ; 7,20 ; 6,96 ; δH₆, H₈, H₉ : 8,10 ; 7,90 ; 7,30 ; δH C₆H₄-F : 7,76 ; 7,30 ; δH NH⁺ : 4,80 ; δH CH₂ : 4,40 ; 4,00 ; 3,33 ; δH OCH₃ : 3,82

### EXEMPLE 10 : 4-(4-BENZYLPIPERAZINO)-7-METHOXY-PYRROLO[1,2-a]QUINOXALINE

### EXEMPLE 11 : 6-(4-BENZYLPIPERAZINO)-PYRIDO[3,2-e]PYRROLO[1,2-a]PYRAZINE

En procédant comme dans l'exemple 1, mais en remplaçant au stade A la 2-(pyrrol-1-yl)aniline par la 3-amino-2-(pyrrol-1-yl)pyridine, on obtient le composé du titre. 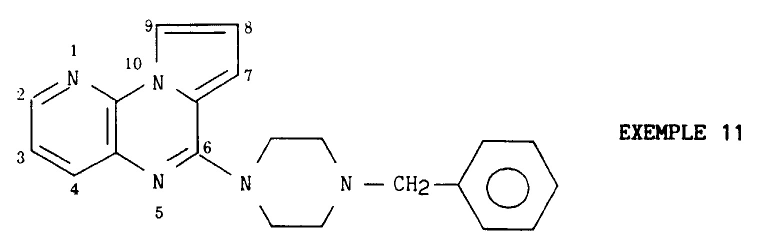
**Stade A : 5,6-dihydro-6-oxo-pyrido[3,2-e]pyrrolo[1,2-a]pyrazine**
   Point de fusion : > 265°C
   Référence : Lancelot et al. Chem. Pharm. Bull. 1985, vol 33, p 2798
**Stade B : 6-chloro-pyrido[3,2-e]pyrrolo[1,2-a]pyrazine**
   Solvant de recristallisation : acétonitrile
   Point de fusion : 146°C
   Rendement : 82 %
   Caractéristiques spectrales :
   Infrarouge : νcm⁻¹ (KBr) : bandes principales à : 1590, 1400, 1260, 1090, 790 et 730.
   Spectre de RMN (DMSOd₆) : δH pyrroliques 8,33 ; 7,03 ; 6,93 ; δ H₂, H₃, H₄ (pyridiniques) : 8,50 ; 8,13 et 7,50
**Stade C : 6-(4-benzylpipérazino)-pyrido[3,2-e]pyrrolo[1,2-a]pyrazine**
   Solvant de recristallisation : acétonitrile
   Point de fusion (trichlorhydrate) : 186°C
   Rendement : 81 %
   Caractéristiques spectrales :
   Infrarouge : νcm⁻¹ (KBr) 2300, 2470, 2540, 2800 NH⁺, bandes principales à : 1600, 1440, 1305, 970, 760 et et 710.
   Spectre de RMN (DMSOd₆) : δH pyrroliques 8,40 ; 7,70 ; 6,96 ; δH₂, H₃, H₄ : 8,40 ; 7,43 ; δH C₆H₅ : 7,70 ; 7,43 ; δH NH⁺ : 5,03 ; δH CH₂ : 4,43 ; 4,16 ; 3,43

### EXEMPLE 12 : 6-[4-(4-FLUOROBENZYL)PIPERAZINO]-PYRIDO[3,2-e]PYRROLO[1,2-a]PYRAZINE

En procédant comme dans l'exemple 11, mais en remplaçant au stade C la 1-benzylpipérazine par la 1-(4-fluorobenzyl)pipérazine, on obtient le composé du titre.
Solvant de recristallisation : acétonitrile
Point de fusion (trichlorhydrate) : 202°C
Rendement : 63 %
Caractéristiques spectrales :
Infrarouge : νcm⁻¹ (KBr) 2560, 2850 NH⁺, bandes principales à : 1610, 1440, 1235, 975, 835 et 770.
Spectre de RMN (DMSOd₆) : δH pyrroliques 8,40 ; 7,20 ; 6,93 ; δH₁, H₂, H₃: 8,40 ; 7,20 ; δH C₆H₄-F : 7,76 ; 7,43 ; δH NH⁺ : 5,10 ; δH CH₂ : 4,43 ; 3,40

### EXEMPLE 13 : 6-(4-ALLYLPIPERAZINO)-PYRIDO[3,2-e]PYRROLO[1,2-a]PYRAZINE

En procédant comme dans l'exemple 11, mais en remplaçant au stade C la 1-benzylpipérazine par la 1-allylpipérazine, on obtient le composé du titre.
Solvant de recristallisation : acétonitrile
Sublimation à 250°C (trichlorhydrate)
Rendement : 23 %
Caractéristiques spectrales.
Infrarouge : νcm⁻¹ (KBr) 2340, 2530 (NH⁺), bandes principales à : 1620, 1450, 1310, 980 et 780.
Spectre de RMN (DMSOd₆) : δH pyrroliques 8,43 ; 7,50 ; 6,96 ; δH₁, H₂, H₃: 8,43 ; 7,50 ; δH(alkyl) : 6,00 ; 5,56 ; δH NH⁺ 4,20 ; δH CH₂ : 4,20 ; 3,90 et 3,43

### EXEMPLE 14 : 6-(4-BENZYLPIPERAZINO)-PYRAZINO[2,3-e]PYRROLO[1,2-a] PYRAZINE

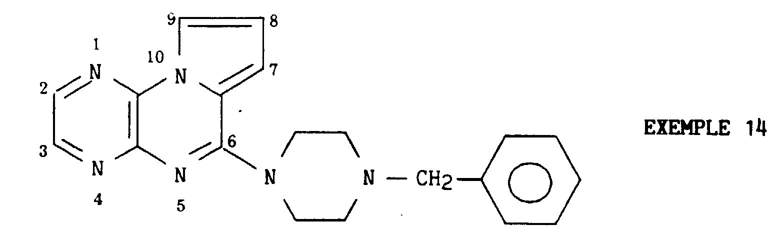

En procédant comme dans l'exemple 1, mais en remplaçant au stade A' l'acide 2-(pyrrol-1-yl)benzoïque par l'acide 3-(pyrrol-1-yl)pyrazinoïque, on obtient le composé du titre.
**Stade A : 5,6-dihydro-6-oxo-pyrazino[2,3-e]pyrrolo[1,2-a]pyrazine**
   Point de fusion : 238°C
   Référence : Lancelot et al. Chem. Pharm. Bull. 1985, vol 33, p 3122
**Stade B : 6-chloro-pyrazino[2,3-e]pyrrolo[1,2-a]pyrazine**
   Solvant de recristallisation : acétonitrile
   Point de fusion : 182°C
   Rendement : 21 %
   Caractéristiques spectrales :
   Infrarouge : νcm⁻¹(KBr) : bandes principales à : 1600, 1585, 1270, 780 et 735.
   Spectre de RMN (DMSOd₆) : δH pyrroliques 8,66 ; 7,06 ; 6,98 ; δH₂, H₃, 8,28 ; 8,13
**Stade C : 6-(4-benzylpipérazino)-pyrazino[2,3-e]pyrrolo[1,2-a]pyrazine**
   Solvant de recristallisation : acétonitrile
   Point de fusion (trichlorhydrate) : 200°C
   Caractéristiques spectrales :
   Infrarouge : νcm⁻¹ (KBr) 2350, 2540 NH⁺, bandes principales à : 1610, 1440, 1330, 970 et 760.
   Spectre de RMN (DMSOd₆) : δH pyrroliques 8,48 ; 7,28 ; 6,96 ; δH₂, H₃ : 8,48 ; δH C₆H₅ : 7,45 ; δNH⁺ : 5,03 ; δH CH₂ : 4,60 ; 4,43 et 3,43.

### EXEMPLE 15 : 6-[4-(4-FLUOROBENZYL)PIPERAZINO]-PYRAZINO[2,3-e]PYRROLO[1,2-a]PYRAZINE

En procédant comme dans l'exemple 14, mais en remplaçant au stade C la 1-benzylpipérazine par la 1-(4-fluorobenzyl)pipérazine, on obtient le composé du titre.

### EXEMPLE 16 : 7-CHLORO-4-[4-(3,4-METHYLENEDIOXYBENZYL)PIPERAZINO]-PYRROLO[1,2-a]QUINOXALINE

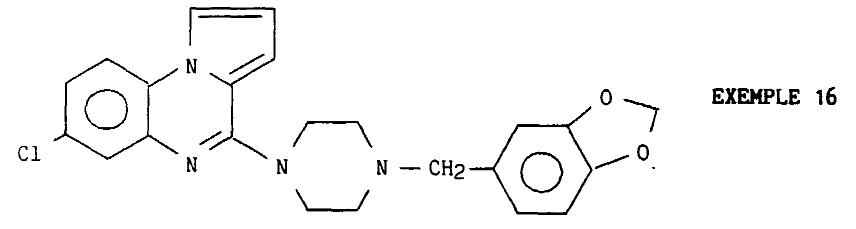

En procédant comme dans l'exemple 3, mais en remplaçant au stade C la 1-benzylpipérazine par la 1-(3,4-méthylènedioxybenzyl)pipérazine, on obtient le composé du titre.
Solvant de recristallisation : acétonitrile
Point de fusion (trichlorhydrate) : 208°C
Rendement : 67 %
Caractéristiques spectrales :
Infrarouge : νcm⁻¹ (KBr) : 2600, 2700 (NH⁺) bandes principales à : 1600, 1270, 1040, 820 et 775.
Spectre de RMN (DMSOd₆) : δH pyrroliques 8,50 ; 6,96 ; δH₆, H₇, H₉ : 8,13 ; 7,36 ; δH 3,4-méthylènedioxybenzyl : 7,36 ; 6,96 ; δH CH₂ : 3,4-méthylènedioxybenzyl : 6,00 ; δH CH₂ : 4,30 ; 4,00 ; 3,36 ; δH NH⁺ : 4,90

### EXEMPLE 17 A 62 :

En suivant les procédés décrits lors des exemples 1 à 9, mais en remplaçant aux stades C la 1-benzylpipérazine ou la 1-(4-fluorobenzyl) pipérazine par les amines appropriées, on obtient les composés des exemples suivants :
**EXEMPLE 17 : 4-[4-(PYRROLIDINOCARBONYLMETHYL)PIPERAZINO]-PYRROLO [1,2-a]QUINOXALINE**
**EXEMPLE 18 : 4-{4-[1-(4-CHLOROPHENYL)-1-(PHENYL)METHYL]PIPERAZINO}-PYRROLO[1,2-a]QUINOXALINE**
**EXEMPLE 19 : 4-(1,2,5,6-TETRAHYDROPYRID-1-YL)-PYRROLO[1,2-a] QUINOXALINE**
**EXEMPLE 20 : 4-(1,4-THIOMORPHOLINO)-PYRROLO[1,2-a]QUINOXALINE**
**EXEMPLE 21 : 4-(4-PHENYLPIPERIDINO)-PYRROLO[1,2-a]QUINOXALINE**
**EXEMPLE 22 : 4-(4-PHENYLPIPERAZINO)-PYRROLO[1,2-a ]QUINOXALINE**
**EXEMPLE 23 : 4-[4-(3-TRIFLUOROMETHYLPHENYL)PIPERAZINO]-PYRROLO[1,2-a]QUINOXALINE**
**EXEMPLE 24 : 4-[4-(ETHOXYCARBONYL)PIPERAZINO]-PYRROLO[1,2-a]QUINOXALINE**
**EXEMPLE 25 : 4-[4-(PYRID-2-YL)PIPERAZINO]-PYRROLO[1,2-a]QUINOXALINE**
**EXEMPLE 26 : 4-[4-(2,4-DICHLOROBENZYL)PIPERAZINO]-PYRROLO[1,2-a] QUINOXALINE**
**EXEMPLE 27 : 4-[4-(3,4-DICHLOROBENZYL)PIPERAZINO]-PYRROLO[1,2-a] QUINOXALINE**
**EXEMPLE 28 : 4-[4-(4-CHLOROPHENYL)PIPERAZINO]-PYRROLO[1,2-a]QUINOXALINE**
**EXEMPLE 29 : 4-[4-(4-FLUOROPHENYL)PIPERAZINO]-PYRROLO[1,2-a]QUINOXALINE**
**EXEMPLE 30 : 4-(4-PROPYLPIPERAZINO)-PYRROLO[1,2-a]QUINOXALINE**
**EXEMPLE 31 : 4-(4-ALLYLPIPERAZINO)-PYRROLO[1,2-a]QUINOXALINE**
**EXEMPLE 32 : 4-{4[4,4-bis(4-CHLOROPHENYL)BUTYL]PIPERAZINO}-PYRROLO[1,2-a]QUINOXALINE**
**EXEMPLE 33 : 4-(2,6-DIMETHYLMORPHOLINO)-PYRROLO[1,2-a]QUINOXALINE**
**EXEMPLE 34 : 4-{[2-(BENZYLAMINO)ETHYL]AMINO}-PYRROLO[1,2-a]QUINOXALINE**
**EXEMPLE 35 : 4-[2,5-DIMETHYLPIPERAZINO]-PYRROLO[1,2-a]QUINOXALINE**
**EXEMPLE 36 : 4-(3,3,5-TRIMETHYLPERHYDROAZEPIN-1-YL)-PYRROLO[1,2-a] QUINOXALINE**
**EXEMPLE 37 : 4-(PERHYDROAZEPIN-1-YL)-PYRROLO[1,2-a]QUINOXALINE**
**EXEMPLE 38 : 4-[4-(4-CHLOROPHENYL)-4-HYDROXYPIPERIDINO]-PYRROLO[1,2-a] QUINOXALINE**
**EXEMPLE 39 : 7-CHLORO-4-(4-METHYLPIPERAZINO)-PYRROLO[1,2-a]QUINOXALINE**
**EXEMPLE 40 : 8-CHLORO-4-(4-METHYLPIPERAZINO)-PYRROLO[1,2-a]QUINOXALINE**
**EXEMPLE 41 : 7-METHYL-4-(4-METHYLPIPERAZINO)-PYRROLO[1,2-a]QUINOXALINE**
**EXEMPLE 42 : 7-METHOXY-4-(4-METHYLPIPERAZINO)-PYRROLO[1,2-a]QUINOXALINE**
**EXEMPLE 43 : 7-METHYL-4-PIPERAZINO-PYRROLO[1,2-a]QUINOXALINE**
**EXEMPLE 44 : 7-CHLORO-4-PIPERAZINO-PYRROLO[1,2-a]QUINOXALINE**
**EXEMPLE 45 : 7-CHLORO-4-[4-(PYRROLIDINOCARBONYLMETHYL)PIPERAZINO]-PYRROLO[1,2-a]QUINOXALINE**
**EXEMPLE 46 : 7-METHOXY-4-[4-(PYRROLIDINOCARBONYLMETHYL)PIPERAZINO]-PYRROLO[1,2-a]QUINOXALINE**
**EXEMPLE 47 : 7-CHLORO-1-{4-[1-(4-CHLOROPHENYL)-1-(PHENYL)METHYL] PIPERAZINO}-PYRROLO[1,2-a]QUINOXALINE**
**EXEMPLE 48 : 8-CHLORO-4-{4-[1-(4-CHLOROPHENYL)1-(PHENYL)METHYL] PIPERAZINO}-PYRROLO[1,2-a]QUINOXALINE**
**EXEMPLE 49 : 7-METHYL-4-(1,2,5,6-TETRAHYDROPYRID-1-YL)-PYRROLO[1,2-a] QUINOXALINE**
**EXEMPLE 50 : 7-CHLORO-4-(1,4-THIOMORPHOLINO)-PYRROLO[1,2-a]QUINOXALINE**
**EXEMPLE 51 : 7-CHLORO-4-(4-PHENYLPIPERIDINO)-PYRROLO[1,2-a]QUINOXALINE**
**EXEMPLE 52 : 7-METHYL-4-(4-PHENYLPIPERIDINO)-PYRROLO[1,2-a]QUINOXALINE**
**EXEMPLE 53 : 7-CHLORO-4-(4-PHENYLPIPERAZINO)-PYRROLO[1,2-a]QUINOXALINE**
**EXEMPLE 54 : 7-METHOXY-4-(4-PHENYLPIPERAZINO)-PYRROLO[1,2-a]QUINOXALINE**
**EXEMPLE 55 : 7-CHLORO-4-[4-(3-TRIFLUOROMETHYLPHENYL)PIPERAZINO]-PYRROLO [1,2-a]QUINOXALINE**
**EXEMPLE 56 : 7-CHLORO-4-[4-(ETHOXYCARBONYL)PIPERAZINO]-PYRROLO[1,2-a] QUINOXALINE**
**EXEMPLE 57 : 4-[4-(2,4-DICHLOROBENZYL)PIPERAZINO]-7-METHYL-PYRROLO [1,2-a]QUINOXALINE**
**EXEMPLE 58 : 7-CHLORO-4-[4-(2,4-DICHLOROBENZYL)PIPERAZINO]-PYRROLO[1,2-a]QUINOXALINE**
**EXEMPLE 59 : 4-[4-(3,4-DICHLOROBENZYL)PIPERAZINO]-7-METHOXY-PYRROLO [1,2-a]QUINOXALINE**
**EXEMPLE 60 : 8-CHLORO-4-[4-(3,4-DICHLOROBENZYL)PIPERAZINO]-PYRROLO[3,2-a]QUINOXALINE**
**EXEMPLE 61 : 7-CHLORO-4-(4-PROPYLPIPERAZINO)-PYRROLO[1,2-a]QUINOXALINE**
**EXEMPLE 62 : 4-(4-ALLYLPIPERAZINO)-7-CHLORO-PYRROLO[1,2-a]QUINOXALINE**

### EXEMPLES 63 A 72 :

En procédant comme dans l'exemple 11, mais en remplaçant au stade C la 1-benzylpipérazine par l'hétérocycle convenablement substitué, on obtient les composés des exemples suivants :
**EXEMPLE 63 : 6-(4-METHYLPIPERAZINO)-PYRIDO[3,2-e]PYRROLO[1,2-a]PYRAZINE**
   Point de fusion : 260 °C
**EXEMPLE 64 : 6-PIPERAZINO-PYRIDO[3,2-e]PYRROLO[1,2-a]PYRAZINE**
   Point de fusion : 265 °C
**EXEMPLE 65 : 6-{4-[1-(4-CHLOROPHENYL)-1-(PHENYL)METHYL]PIPERAZINO}-PYRIDO[3,2-e]PYRROLO[1,2-a]PYRAZINE**
**EXEMPLE 66 : 6-[4-(ETHOXYCARBONYL)PIPERAZINO]-PYRIDO[3,2-e]PYRROLO[1,2-a]PYRAZINE**
   Point de fusion : 196 °C
**EXEMPLE 67 : 6-(4-PHENYLPIPERIDINO)-PYRIDO[3,2-e]PYRROLO[1,2-a]PYRAZINE**
**EXEMPLE 68 : 6-(4-PHENYLPIPERAZINO)-PYRIDO[3,2-e]PYRROLO[1,2-a]PYRAZINE**
   Point de fusion : 180 °C
**EXEMPLE 69 : 6-[4-(2,4-DICHLOROBENZYL)PIPERAZINO]-PYRIDO[3,2-e]PYRROLO [1,2-a]PYRAZINE**
   Point de fusion : 220 °C
**EXEMPLE 70 : 6-[4-(3,4-DICHLOROBENZYL)PIPERAZINO]-PYRIDO[3,2-e]PYRROLO [1,2-a]PYRAZINE**
**EXEMPLE 71 : 6-[4-(4-CHLOROPHENYL)PIPERAZINO]-PYRIDO[3,2-e]PYRROLO[1,2-a]PYRAZINE**
**EXEMPLE 72 : 6-(4-PROPYLPIPERAZINO)-PYRIDO[3,2-e]PYRROLO[1,2-a]PYRAZINE**
**EXEMPLE 73 : 6-(4-BENZYLPIPERAZINO)-PYRIMIDINO[4,5-e]PYRROLO[1,2-a] PYRAZINE**
**EXEMPLE 74 : 6-[4-(4-FLUOROBENZYL)PIPERAZINO]-PYRIMIDINO[4,5-e]PYRROLO [1,2-a]PYRAZINE**

### EXEMPLES 75 A 79 :

En procédant comme dans l'exemple 1, mais en remplaçant au stade C la 1-benzylpipérazine par les amines appropriées, on obtient les composés des exemples suivants :
**EXEMPLE 75 : 4-[2-(N,N-DIMETHYLAMINO)ETHYLAMINO]-PYRROLO[1,2-a] QUINOXALINE**
**EXEMPLE 76 : 4-[4-(3-PHENYLPROP-2-EN-1-YL)PIPERAZINO]-PYRROLO[1,2-a] QUINOXALINE**
**EXEMPLE 77 : 4-{4-[2-(2-TRIFLUOROMETHYLPHENYL)ETHYL]PIPERAZINO}-PYRROLO [1,2-a]QUINOXALINE**
**EXEMPLE 78 : 4-MORPHOLINO-PYRROLO[1,2-a]QUINOXALINE**
**EXEMPLE 79 : 4-PYRROLIDINO-PYRROLO[1,2-a]QUINOXALINE**
**EXEMPLE 80 : 4-{4-{2-[(METHYLAMINO)SULFONYL]ETHYL}PIPERAZINO}-PYRROLO [1,2-a]QUINOXALINE**

### EXEMPLES 81 A 87 :

En procédant comme dans l'exemple 11, mais en remplaçant au stade C la 1-benzylpipérazine par les amines appropriées, on obtient les composés des exemples suivants :
**EXEMPLE 81 : 6-[2-(N,N-DIMETHYLAMINO)ETHYLAMINO]-PYRIDO[3,2-e]PYRROLO [1,2-a]PYRAZINE**
**EXEMPLE 82 : 6-[4-(3-PHENYLPROP-2-EN-1-YL)PIPERAZINO]-PYRIDO[3,2-e] PYRROLO[1,2-a]PYRAZINE**
**EXEMPLE 83 : 6-{4-[2-(2-TRIFLUOROMETHYLPHENYL)ETHYL]PIPERAZINO}-PYRIDO [3,2-e]PYRROLO[1,2-a]PYRAZINE**
**EXEMPLE 84 : 6-MORPHOLINO-PYRIDO[3,2-e]PYRROLO[1,2-a]PYRAZINE**
**EXEMPLE 85 : 6-PYRROLIDINO-PYRIDO[3,2-e]PYRROLO[1,2-a]PYRAZINE**
**EXEMPLE 86 : 6-THIOMORPHOLINO-PYRIDO[3,2-e]PYRROLO[1,2-a]PYRAZINE**
**EXEMPLE 87 : 6-{4-{2-[(METHYLAMINO)SULFONYL]ETHYL}PIPERAZINO}-PYRIDO [3,2-e]PYRROLO[1,2-a]PYRAZINE**
**EXEMPLE 88 : 8-BENZYLOXY-4-PIPERAZINO-PYRROLO[1,2-a]QUINOXALINE**
**EXEMPLE 89 : ACIDE 3-[4-(8-BENZYLOXY-PYRROLO[1,2-a]QUINOXALIN-4-YL)PIPERAZINO]PROPIONIQUE**
**EXEMPLE 90 : TRICHLORHYDRATE DE 2-METHOXY-6-(4-BENZYLPIPERAZINO)PYRIDO [3,2-e]PYRROLO[1,2-a]PYRAZINE**

**Stade A : 2-amino-6-méthoxy-3-nitropyridine**
   1,65 g (0,0717 mol ; 2 équivalents (éq)) de sodium sont dissous dans 100 cm3 de méthanol. 6,2 g (35,7 mmol) de 2-amino-6-chloro-3-nitropyridine sont ajoutés et la solution est portée au reflux pendant 8h. Le méthanol est évaporé sous pression réduite et le résidu est repris par un minimum d'eau (20 cm³). La solution est extraite par 100 cm³ d'éther éthylique. La phase éthére est lavée à l'eau (20 cm³), décantée, séchée sur MgSO₄, décolorée au charbon animal et évaporée au rotavapor pour donner un 1er jet. La phase aqueuse est extraite dans les mêmes conditions par 2 fois 50 cm³ d'acétate d'éthyle donnant un 2ème et 3ème jet.
   On obtient 4,7 g (0,0278 mol ; rendement : 77,7 %) d'une poudre jaune.
   Point de fusion : 172°C
**Stade B : 6-méthoxy-3-nitro-2-(pyrrol-1-yl)-pyridine**
   10,45 g (0,0791 mol ; 1,2 éq) de 2,5-diméthoxytétrahydrofurane et 12,00 g (0,0791 mol ; 1,2 éq) de chlorhydrate de 4-chloropyridine sont agités 10 minutes dans 300 cm³ de dioxane. 11,15 g (0,0659 mol) de 2-amino-6-méthoxy-3-nitropyridine sont ajoutés et la solution est portée aux reflux 4 h. Le dioxane est évaporé sous pression réduite.
   Le résidu est repris dans 250 cm³ d'eau puis extrait par 500 cm³ d'éther éthylique. La phase éthérée est lavée à l'eau, décantée, séchée sur MgSO₄, décolorée au charbon animal et concentrée au rotavapor.
   On obtient 11,12 g (0,0507 mol) d'une poudre orange.
   Rendement : 76,9 %
   Point de fusion 70°C
**Stade C : 3-amino-6-méthoxy-2-(pyrrol-1-yl)-pyridine**
   11,12 g (0,0507 mol) de 6-mothoxy-3-nitro-2-(pyrrol-1-yl)-pyridine sont dissous dans 300 cm³ d'éthanol absolu.
   5 spatules de Nickel de Raney (environ 8 g) sont ajoutés puis 2 cm³ d'hydrazine monohydratée.
   La solution est chauffée doucement 30 minutes puis 28 cm³ d'hydrazine monohydratée (en tout 0,617 mol ; 12,1 éq) sont ajoutés goutte à goutte en 2h. La solution est au reflux pendant cette période et pendant 3h supplémentaires. Après complet refroidissement, la solution est filtrée puis concentrée sous pression réduite pour donner une huile brune. Le précipité Nickel de Raney est neutralisé par de l'HCl 10 N.
   L'huile peut être directement engagée dans l'étape suivante.
   On obtient 8,21 g (0,0434 mol ; rendement : 85,5 %) d'huile.
**Stade D : 5,6-dihydro-2-méthoxy-6-oxo-pyrido[3,2-e]pyrrolo[1,2-a]pyrazine**
   8,21g (0,0434 mol) de 3-amino-6-méthoxy-2-(pyrrol-1-yl)-pyridine sont dissous dans 400 cm³ de toluène. 30 cm³ (0,0576 mol ; 1,3 éq) de phosgène à 20 % en solution toluénique sont additionnés et la suspension est portée au reflux 3h. La suspension est passée sous un courant d'azote pendant 1h. Le précipité formé est essoré sur verre fritté et donne le dérivé attendu sous forme chlorhydrate (Point de Fusion : 245°C). Il est repris et alcalinisé par une solution saturée de NaHCO₃. Le précipité résiduel est éssoré sur verre fritté pour donner 3,4 g du produit désiré. Le filtrat toluénique est concentré sous pression réduite ; le résidu est repris par 100 cm³ d'éther éthylique puis éssoré sur verre fritté. Le précipité est traité par une solution saturée de NaHCO₃ puis essoré sur verre fritté pour donner 3,56 g de produit final.
   On obtient en tout 6,96 g (0,323 mol ; rendement : 74,5 %) de poudre crème.
   Point de fusion : 250°C
**Stade E : 6-chloro-2-méthoxy-pyrido[3,2-e]pyrrolo[1,2-a]pyrazine**
   2,6 g (0,0120 mol) de 5,6-dihydro-2-méthoxy-6-oxopyrido[3,2-e]pyrrolo[1,2-a]pyrazine sont ajoutés à une solution de 80 cm³ d'oxychlorure de phosphore et de 4 cm³ de pyridine. La solution est portée au reflux 5h. Après refroidissement, l'oxychlorure de phosphore est concentré sous pression réduite. Le résidu est repris par 100 cm³ d'eau glacée puis est doucement alcalinisée par une solution d'ammoniaque à 33 %. Le précipité est éssoré sur verre fritté. Il est extrait par 150 cm³ d'éther éthylique puis par 100 cm³ d'acétate d'éthyle. Les phases organiques sont lavées à l'eau, séchées sur MgSO₄ et concentrées au rotavapor.
   On obtient 0,94 g (0,0040 mol) d'une poudre jaune.
   Rendement : 33,2 %
   Point de fusion : 163°C
**Stade F : 6-(4-benzylpipérazino)-2-méthoxy-pyrido[3,2-e]pyrrolo[1,2-a]pyrazine**
   0,70 g (0,00397 mol ; 1,1 éq) de 1-benzylpipérazine et 0,60 g (0,00434 mol ; 1,2 éq) de K₂CO₃ sont ajoutés à 20 cm³ de diméthylformamide (DMF). 0,84 g (0,00359 mol) de 6-chloro-2-méthoxypyrido[3,2-e]pyrrolo[1,2-a]pyrazine sont ajoutés et la solution est portée au reflux 3h. Après refroidissement, la solution est versée sur 100 cm³ d'eau agitée puis extraite avec 300 cm³ d'éther éthylique. La phase éthérée est lavée à l'eau, séchée sur MgSO₄, décolorée au charbon animal puis concentré au rotavapor pour donner la base. Celle-ci est dissoute à 60°C dans 100 cm³ d'isopropanol puis on ajoute rapidement à température ordinaire 4 cm³ d'HCl 10 N. Après 2 h d'agitation, le précipité est essoré sur verre fritté puis rincé avec 50 cm³ d'isopropanol et rincé avec 50 cm³ d'éther éthylique anhydre.
   On obtient 0,70 g (0,00143 mol, rendement : 40,3 %) d'une poudre blanche.
   Point de fusion (trichlorhydrate) : > 264°C (acétonitrile/isopropanol : 100/40)
   IR(KBr νcm⁻¹) 3360(NH⁺) ; 1610 ; 1575 ; 1480 ; 1270 ; 995 ; 695.
   RMN¹H(DMSO-d₆) δ : 8,30 (m, 2H, H₄H₉) 7,36-7,43(m, 6H, C₆H₅, H₃) 6,93(m, 2H, H₇H₈) 4,43-3,42(m, 13H ; CH₂ NH⁺) 3,98(s, 3H, CH₃) C₂₂ H₂₃ N₅ O ; 3HCl (PM : 482, 822).

### EXEMPLE 91 : 6-[4-(4-FLUOROBENZYL)PIPERAZINO)-2-METHOXY-PYRIDO[3,2-e]PYRROLO[1,2-a]PYRAZINE

En procédant de la même façon que dans l'exemple 90 mais en utilisant au stade F 2,00 g (0,00855 mol) de 6-chloro-2-méthoxypyrido[3,2-e] pyrrolo[1,2-a]pyrazine, 1,66 g (0,00585 mol) de 1-(4-fluorobenzyl)pipérazine, 1,30 g (0,00941 mol ; 1,1 éq) de K₂CO₃ et 30 ml de DMF, on obtient le composé du titre.
L'extraction se fait par 400 cm³ d'éther éthylique, et le sel est obtenu avec 4 cm³ d'HCl 10N/isopropanol.
On obtient 1,25 g (0,00269 mol, rendement : 31,4 %) d'une poudre jaune clair.
Point de fusion (dichlorhydrate) : 210°C (acétonitrile)
IR(KBr νcm⁻¹) 3380(NH⁺) 2950 ; 1610 ; 1575 ; 1470 ; 1275 ; 990 ; 750. RMN¹H(DMSO-d₆) δ : 8,40 - 8,28 (m, 2H, H₄H₉) 7,75-7,37-7,25(m, 5H, C₆H₄, H₃) 6,91-6,78(m, 2H, H₇H₈) 4,85(m, NH⁺ + CH₂) 4,47(m, 4H, CH₂ pipé) 3,95(s, 3H, CH₃) 3,45(m, 4H, CH₂ pipé) C₂₂ H₂₂ F N₅ O ; 2 HCl (464, 353).

### EXEMPLE 92 : 2-BENZYLOXY-6-(4-BENZYLPIPERAZINO)PYRIDO[3,2-e]PYRROLO[1,2-a]PYRAZINE

**Stade A : 2-amino-6-benzyloxy-3-nitropyridine**
   1,64 g (0,0713 mol ; 1,2 éq) de sodium sont mis dans 150 cm³ de toluène puis 7,73 g (0,0713 mol ; 1,2 éq) d'alcool benzylique. Après totale dissolution, 10,34 g (0,0595 mol) de 2-amino-6-chloro-3-nitropyridine sont ajoutés et le chauffage est progressivement augmenté en 30 minutes puis la solution est mise au reflux 3h. Après refroidissement, 50 cm³ d'eau sont ajoutés et le toluène est éliminé sous pression réduite. La solution résiduelle est extraite par 400 cm³ d'éther éthylique. La phase éthérée est décantée, séchée sur MgSO₄, décolorée au charbon animal et concentrée au rotavapor.
   Le résidu semi-solide est repris par 20 cm³ d'éther éthylique et essoré sur verre fritté où le produit solide est récupéré.
   On obtient 6,73 g (0,0274 mol ; rendement 46,0 %) d'une poudre orange.
   Point de fusion : 134° C (éther éthylique).
**Stade B : 6-benzyloxy-3-nitro-2-pyrrol-1-yl-pyridine**
   8,2 g (0,062 mol ; 1,2 éq) de 2,5-diméthoxytétrahydrofurane sont agités 10 minutes dans 200 cm³ d'acide acétique glacial. 12,68 g (0,0517 mol) de 2-amino-6-benzyloxy-3-nitropyridine sont ajoutés et la solution est portée au reflux 4 h, l'acide acétique est éliminé sous pression réduite. Le résidu est alcalinisé par 200 cm³ d'une solution saturée de NaHCO₃ puis extrait par 400 cm³ d'éther éthylique. La phase éthérée est lavée à l'eau, séchée sur MgSO₄, décolorée au charbon animal et concentrée sous pression réduite.
   On obtient 8,48 g (0,0287 mol ; rendement 55,5 %) d'une poudre rouge.
   Point de fusion : 130°C (éther éthylique)
**Stade C** : **3-amino-6-benzyloxy-2-pyrrol-1-yl-pyridine**
   En procédant de la même façon qu'au stade C de l'exemple 90, mais en utilisant 7,8 g (0,0264 mol) de 6-benzyloxy-3-nitro-2-pyrrol-1-ylpyridine, 4 spatules (environ 7 g) de Nickel de Raney et 10 cm³ (0,206 mol ; 7,8 éq) d'hydrazine monohydratée, on obtient 6,41 g (0,0241 mol rendement : 91,3 %) d'une huile brune correspondant au composé du titre.
**Stade D : 5,6-dihydro-2-benzyloxy-6-oxo-pyrido[3,2-e]pyrrolo[1,2-a]pyrazine**
   En procédant de la même façon qu'au stade D de l'exemple 90, mais sans concentrer le filtrat toluénique et en utilisant 6,04 g (0,0241 mol) de 3-amino-6-benzyloxy-2-pyrrol-1-yl-pyridine, 15 cm³ (0,0288 mol ; 1,2 éq) de COCl₂/toluène et 250 cm³ de toluène, on obtient 2,73 g (0,0094 mol, rendement : 38,8 %) d'une poudre beige.
   Point de fusion : 264°C
**Stade E : 2-benzyloxy-6-chloro-pyrido[3,2-e]pyrrolo[1,2-a]pyrazine**
   En procédant de la même façon qu'au stade E de l'exemple 90, mais en utilisant 2,65 g (0,0091 mol) de 5,6-dihydro-2-benzyloxy-6-oxo-pyrido[3,2-e]pyrrolo[1,2-a]pyrazine, 10 cm³ de pyridine et 100 cm³ de POCl₃, on obtient 1,47 g (0,0047 mol, Rendement : 52,1 %) d'une poudre marron.
   Point de fusion : 106°C (acétate d'éthyle).
**Stade F** : **2-benzyloxy-6-(4-benzylpipérazino)-pyrido[3,2-e]pyrrolo[1,2-a]pyrazine**
   En procédant de la même façon qu'au stade F de l'exemple 90, mais en utilisant 1,00 g (0,00322 mol) de 2-benzyloxy-6-chloro-pyrido[3,2-e] pyrrolo[1,2-a]pyrazine et 0,54 g (0,00387 mol ; 1,2 éq) de K₂CO₃, on obtient le composé du titre.
   Pour former le sel, la base est reprise dans 60 cm³ d'acétone ; 1,1 éq d'acide fumarique (0,41 g) est ajouté et la suspension est chauffée 15 minutes. Après refroidissement, le précipité est essoré sur verre fritté puis rincé avec 30 cm³ d'éther éthylique anhydre. On obtient 0,88 g (0,0015 mol, rendement 48,1 %) d'une poudre blanche.
   Point de fusion (monofumarate) : 208°C (acétonitrile)
   IR(KBᵣ νcm⁻¹) 1670(CO) ; 1430 ; 1270 ; 1230 ; 1010 ; 930 ; 650.
   RMN¹H(DMSO d₆) δ : 7,80 (t, 1H, H₉) 7,47(d, 1H, H₄) 7,00(m, 10H, 2 C₆H₅) 6,60-6,50(m, 3H ; H₃ H₇ H₈) 6,27(s, 2H, CH fumarate) 5,27 (s, 2H, CH₂O) 4,20 (s,2H, CH₂) 3,40-2,63-2,17(m, 8H, CH₂ pipérazine) C₂₈ H₂₇ N₅ O, C₄H₄O₄ (565, 603)

### EXEMPLE 93 : 2,6-BIS-(4-BENZYLPIPERAZINO)-PYRIDO[3,2-e]PYRROLO[1,2-a]PYRAZINE

**Stade A : 6-chloro-3-nitro-2-pyrrol-1-yl-pyridine**
   On procède de la même façon qu'au stade B de l'exemple 90, mais on part de 15,40 g (0,0887 mol) de 6-chloro-3-nitro-2-amino-pyridine, et en utilisant 12,9 g (0,0976 mol ; 1,1 éq) de 2,5-diméthoxytétrahydrofurane, 14,64 g (0,0976 mol ; 1,1 éq) de chlorhydrate de 4-chloropyridine et 300 cm³ de 1,4-dioxane ; la solution est portée au reflux 6h. En sortie de réaction, le précipité est repris dans l'éther éthylique.
   L'insoluble est filtré (Point de fusion :192°C), la solution éthérée est concentrée sous pression réduite pour fournir le dérivé attendu.
   On obtient 10,27 g (0,0459 mol, rendement 51,7 %) d'une poudre rouge.
   Point de fusion : 110°C
**Stade B : 3-amino-6-chloro-2-pyrrol-1-yl-pyridine**
   9,27 g (0,0414 mol) de 6-chloro-3-nitro-2-pyrrol-1-yl-pyridine sont dissoutes dans 300 cm³ d'éthanol à 95 %. 115,25 g (0,414 mol ; 10 éq) de sulfate de fer heptahydraté sont ajoutés puis 0,5 cm³ d'HCl 10 N et enfin 5 cm³ d'eau. La solution est portée à reflux en ajoutant pendant les 20 premières minutes 57 cm³ de solution d'ammoniaque pour maintenir le pH supérieur à 7.
   Le reflux est maintenu 1h en ajoutant 6 cm³ d'ammoniaque.Après refroidissement, l'éthanol est éliminé au rotavapor. Le résidu est repris par 100 cm³ d'eau et extrait par 200 cm³ d'éther éthylique. La phase éthérée est lavée à l'eau, décantée, séchée sur MgSO₄, décolorée au charbon animal et concentrée au rotavapor pour donner un 1er jet.
   La phase aqueuse alcalinisée par NH₄OH est extraite par 200 cm³ d'acétate d'éthyle. La phase organique traitée de la même façon que la phase éthérée donne un 2ème jet.
   On obtient 4,59 g (0,0237 mol ; rendement : 57, 1 %) d'une poudre beige.
   Point de fusion : 89°C (éther éthylique/hexane : 80/20).
**Stade C** : **5,6-dihydro-2-chloro-6-oxo-pyrido[3,2-e]pyrrolo[1,2-a]pyrazine**
   En procédant de la même façon qu'au stade D de l'exemple 90, mais sans concentrer le filtrat toluénique, et en utilisant 4,59 g (0,0237 mol) de 3-amino-6-chloro-2-pyrrol-1-yl-pyridine, 12,5 cm³ (0,0240 mol ; 1 éq) de COCl₂/toluène et 200 cm³ de toluène, on obtient 2,6 g (0,0118 mol) de poudre grise
   Rendement : 49, 9 %
   Point de fusion : 270°C
**Stade D : 2,6-dichloro-pyrido[3,2-e]pyrrolo[1,2-a]pyrazine**
   En procédant de la même façon qu'au stade E de l'exemple 90, mais en partant de 2,6 g (0,0118 mol) de 5,6-dihydro-2-chloro-6-oxo-pyrido[3,2-e]pyrrolo[1,2-a]pyrazine, et en utilisant 100 cm³ de POCl₃ et 7 cm³ de pyridine, on obtient 1,27 g (0,00533 mol, rendement : 45,0 %) d'une poudre jaune.
   Point de fusion : 211°C (acétate d'éthyle).
**Stade E : 2,6-bis-(4-benzylpipérazino)-pyrido-[3,2-e]pyrrolo[1,2-a]pyrazine**
   En procédant de la même façon qu'au stade F de l'exemple 90, mais en partant de 1,30 g (0,00 546 mol) de 2,6-dichloropyrido-[3,2-e]pyrrolo[1,2-a]pyrazine, et en utilisant 2,12 g (0,0120 mol ; 2,2 éq) de 1-benzylpipérazine, 1,89 g (0,0137 mol ; 2,5 éq) de K₂CO₃ et 30 cm³ de DMF, et un portant la solution à reflux 4 h, on obtient 1,37 g (0,00206 mol, rendement : 37,8 %) de poudre jaune.
   Point de fusion (tétrachlorhydrate) : 210°C (acétonitrile)
   IR(KBᵣ νcm⁻¹) 3350 ; 1470 ; 1150 ; 1050 ; 730 ; 700.
   Spectre de masse : Pie moléculaire à 517 (M/e) ; C₃₂ H₃₅ N₇, 4 HCl (663, 493)
   RMN¹H(DMSO-d₆) Produit insoluble dans le DMSO

### EXEMPLE 94 : 6-(4-BENZYLPIPERAZINO)-4-METHYL-PYRIDO[3,2-e]PYRROLO[1,2-a]PYRAZINE

**Stade A : 4-méthyl-3-nitro-2-pyrrol-1-yl-pyridine**
   En procédant de la même façon qu'au stade B de l'exemple 92, mais en partant de 9,85 g (0,0643 mol) de 4-méthyl-3-nitro-2-amino-pyridine et en utilisant 10,2 g (0,0772 mol ; 1,2 éq) de 2,5-diméthoxytétrahydrofurane et 200 cm³ de CH₃CO₂H glacial, et en portant la solution au reflux 3 h, on obtient 11,82 g (0,0581 mol, rendement : 90,4 %) d'une poudre jaune rougissant à l'air.
   Point de fusion : 63°C
**Stade B : 3-amino-4-méthyl-2-pyrrol-1-yl-pyridine**
   En procédant de la même façon qu'au stade C de l'exemple 90, mais en partant de 11,82 g (0,0581 mol) de 4-méthyl-3-nitro-2-pyrrol-1-yl-pyridine et en utilisant 5 spatules (≃ 8 g) de Nickel de Raney, 20 cm³ (0,411 mol, 7 éq) d'hydrazine monohydratée et 300 cm³ d'éthanol, on obtient 8,73 g (0,0500 mol ; rendement : 86,6 %) d'une poudre verte.
   Point de fusion : 77°C (éther éthylique)
**Stade C** : **5,6-dihydro-4-méthyl-6-oxo-pyrido[3,2-e]pyrrolo[1,2-a]pyrazine**
   En procédénat de la même façon qu'au stade D de l'exemple 92, mais en partant de 8,73 g (0,050 mol) de 3-amino-4-méthyl-2-pyrrol-1-yl-pyridine et en utilisant 30 cm³ (0,0576 mol, 1,15 éq) de COCl₂/toluène et 250 cm³ de toluène, on obtient 7,8 g (0,0392 mol, rendement : 77,6 %) d'une poudre beige.
   Point de fusion : > 264°C
**Stade D : 6-chloro-4-méthyl-pyrido[3,2-e]pyrrolo[1,2-a]pyrazine**
   En procédant de la même façon qu'au stade E de l'exemple 90, mais en partant de 7,8 g (39,2 × 10⁻³ mol) de 5,6-dihydro-4-méthyl-6-oxo-pyrido [3,2-e]pyrrolo[1,2-a]pyrazine et en utilisant 150 cm³ de POCl₃ et 10 cm³ de pyridine, on obtient 5,83 g (0,0268 mol) d'une poudre jaune.
   Rendement : 68,4 %
   Point de fusion : 139°C (acétate d'éthyle)
**Stade E : 6-(4-benzylpipérazino)-4-méthyl-pyrido[3,2-e]pyrrolo[1,2-a]pyrazine**
   En procédant de la même façon qu'au stade F de l'exemple 90, mais en partant de 2,00 g (0,0092 mol) de 6-chloro-4-méthyl-pyrido[3,2-e]pyrrolo[1,2-a] pyrazine et en utilisant 1,78 g (0,0101 mol ; 1,1 éq) de 1-benzylpipérazine, 1,52 g (0,011 mol ; 1,2 éq) de K₂CO₃ et 30 cm³ de DMF, on extrait la solution par de l'éther éthylique puis de l'acécate d'éthyle.
   On obtient 3,00 g (0,00697 mol ; rendement 75,8 %) d'une poudre jaune
   Point de fusion (dichlorhydrate) : 240°C (acétonitrile/isopropanol : 60-40)
   IR(KBᵣ νcm⁻¹) 3400(NH⁺) ; 3080, 2440, 1480, 1430, 1260, 1110, 955, 830 : RMN¹H(DMSO-d₆) δ : 8,30 (t, 1H, H₉) 8,18(d, 1H, H₂) 7,67-7,46(m, 5H, C₆H₅) 7,30(d, 1H ; H₃) 7,10(t, 1H, H₈) 6,83 (t, 1H, H₇) 4,28-3,40 (m, 10H, CH₂) 2,60(s, 3H, CH₃) 11,93(m, NH⁺)
   C₂₂ H₂₃ N₅ ; 2HCl (430, 461)

### EXEMPLE 95 : 6-[4-(4-FLUOROBENZYL)PIPERAZINO]-4-METHYL-PYRIDO[3,2-e]PYRROLO[1,2-a]PYRAZINE

En procédant de la même façon que pour l'exemple 94, mais en utilisant au stade E 1,96 g (0,0101 mol ; 1,1 éq) de 1-(4-fluorobenzyl)pipérazine, 1,52 g (0,011 mol ; 1,2 éq) de K₂CO₃ et 30 cm³ de DMF, puis en extrayant la solution par l'éther éthylique puis par l'acétate d'éthyle, on obtient 3,34 g (0,00745 mol ; rendement : 81,0 %) d'une poudre jaune.
Point de fusion (dichlorhydrate) : 228°C
IR(KBᵣ νcm⁻¹) 3440(NH⁺) ; 1505 ; 1435 ; 1260 ; 1160, 950.
RMN¹H(DMSO-d₆) δ : 8,27 (t, 1H, H₉) 8,13(d, 1H, H₂) 7,73(t, 2H, C₆H₄) 7,27(m, 3H ; H₃ C₆H₄) 7,06(t, 1H, H₈) 6,80(t, 1H, H₇) 5,40(s, NH⁺) 4,40-3,70-3,36(m, 10H, CH₂) 2,56(s, 3H, CH₃)
C₂₂ H₂₂ F N₅ ; 2HCl (448, 353)

### EXEMPLE 96 : 6-[4-(3,4-METHYLENEDIOXYBENZYL)PIPERAZINO]-PYRIDO[3,2-e] PYRROLO[1,2-a]PYRAZINE

En procédant de la même façon qu'au stade F de l'exemple 90, mais en partant de 1,00 g (0,00491 mol) de 6-chloro-pyrido[3,2-e]pyrrolo[1,2-a]pyrazine décrit au stade B de l'exemple 11, en utilisant 1,19 g (0,0054 mol ; 1,1 éq) de N-(3,4-méthylènedioxybenzyl)pipérazine, 0,82 g (0,059 mol ; 1,2 éq) de K₂ CO₃ et 30 cm³ de DMF, on obtient 1,13 g (0,00227 mol ; rendement : 46,3 %) d'une poudre blanche.
Point de fusion (trichlorhydrate) : 220°C
IR(KBᵣ νcm⁻¹) 3400(NH⁺) ; 1605 ; 1475 ; 1425 ; 1290 ; 1250, 1030 ; 930, 770.
RMN¹H(DMSO-d₆) δ : 8,40 (m, 3H, H₂ H₉ H₄) 7,33-6,97(m, 6H, H₃ H₇ H₈ C₆H₃) 6,03(s, 2H, CH₂O) 5,27(m, NH⁺) 4,65-4,33-3,98-3,43(m, 10H, CH₂) C₂₂ H₂₁ N₅ O₂; 3HCl (496, 806)

### EXEMPLE 97 : 4-(PERHYDRO-1,4-DIAZEPIN-1-YL)PYRROLO[1,2-a]QUINOXALINE

1,6 g (0,00789 mol) de 4-chloro-pyrrolo[1,2-a]quinoxaline 7,9 g (0,00789 mol, 10 éq) d'homopipérazine sont finement pulvérisés puis sont introduits dans un ballon à 180°C. Le milieu réactionnel est maintenu au reflux 5 h. 100 cm³ d'eau sont alors ajoutés avec précaution et le ballon est refroidi dans un bain de glace. Le précipité formé est essoré sur verre fritté puis repris par 200 cm³ d'éther éthylique. La phase éthérée est lavée à l'eau, décantée, séchée sur MgSO₄, décolorée au charbon animal et concentrée sous pression réduite pour donner la base.
Celle-ci est reprise dans 100 cm³ d'isopropanol à 60°C. 3 cm³ d'HCl 10N sont rapidement ajoutés goutte à goutte à 25°C. Après 1h d'agitation, le précipité est essoré sur verre fritté, rincé avec 40 cm³ d'isopropanol puis avec 40 cm³ d'éther éthylique anhydre.
On obtient 0,74 g (0,00207 mol, rendement : 26,2 %) d'une poudre blanche.
Point de fusion (dichlorhydrate) : 196°C (acétonitrile)
IR(KBᵣ νcm⁻¹) 3030-2740(NH⁺) ; 1510 ; 1430 ; 1150, 930 ; 730.
C₁₆ H₁₈ N₄ ; 2HCl ; H₂O (357, 270)

### ETUDE PHARMACOLOGIQUE DES COMPOSES DE L'INVENTION

### EXEMPLE A : ETUDE DE LIAISON AUX RECEPTEURS DES COMPOSES DE L'INVENTION

### A-1 : Etude de liaison aux récepteurs sérotoninergiques 5-HT₃

La liaison des composés de l'invention aux récepteurs 5-HT₃ a été déterminée selon les techniques standards de mesure d'affinité.

### PROTOCOLE :

On utilise comme radioligand le BRL 43694, comme ligand non spécifique : l'ICS 205 930 à 10⁻⁵ M et comme tissus les noyaux du tractus solitaire (NTS) et les cellules NG 180-15.

### A-2 : Etude de la liaison des composés de l'invention aux récepteurs sérotoninergiques 5-HT_{1A}, 5-HT_{1B}, 5-HT_{1C}, 5-HT_{1D} et 5HT₂

La liaison des composés de l'invention est mesurée, selon les méthodes conventionnelles
- pour les récepteurs 5-HT_{1A}, par le déplacement de 8-OH-DPAT dans des homogénats de cortex frontal et d'hippocampe de porc,
- pour les récepteurs 5-HT_{1B}, par le déplacement de 5-hydroxytryptamine des homogénats de cortex, striatum, et globus pallidus de rat,
- pour les récepteurs 5-HT_{1C}, par le déplacement de N-méthyl mésulergine et 10⁻⁶ M de spipérone dans des homogénats de plexus choroïde de porc,
- pour les récepteurs 5-HT_{1D}, par le déplacement de 5-OH-tryptamine dans des homogénats de cortex, de striatum et de globus pallidus du porc,
- pour les récepteurs 5-HT₂, par le déplacement de kétansérine dans des homogénats de cortex frontal de veau.

### CONCLUSION :

Il apparaît que les composés de l'invention possèdent une très haute affinité pour les récepteurs 5-HT₃. Les composés de l'invention s'avèrent également posséder une haute sélectivité pour les récepteurs 5-HT₃ par rapport aux autres récepteurs sérotoninergiques sur lesquels ils ne se fixent pas.

### EXEMPLE B : MESURE DE L'ACCUMULATION DE 14C-GUANIDINIUM DANS LES CELLULES NG 108-15

L'accumulation de 14C-guanidinium dans les cellules NG 108-15 est mesurée et permet d'étudier l'interaction des composés testés avec les récepteurs 5-HT₃, sachant que cette accumulation est stimulée par les agonistes des récepteurs 5-HT₃.

Le clone hybride (neuroblastome-gliome) NG 108-15 est cultivé dans les conditions standards (milieu de Dulbecco auquel on ajoute 40 mM de bicarbonate de sodium, 1,8 mM de L-glutamine, 0,1 mM d'hypoxanthine, 1 µM d'aminoptérine, 16 µM de thymine et 10% de sérum de veau foetal) à 37°C dans une atmosphère enrichie en CO₂ (7%). A confluence (≃ 3,5 × 10⁵ cellules dans des boites de 35 mm de diamètre), le milieu de culture est aspiré et le tapis de cellules est lavé rapidement avec 3 cm³ d'un tampon HEPES (20 mM) contenant 145 mM de NaCl, 5,4 mM de KCl, 1,8 mM de CaCl₂, 1,0 mM de MgCl₂ et 20 mM de glucose, pH 7,4. Après élimination de la solution de lavage, on verse doucement dans chaque boîte de culture 1,5 cm³ du même tampon (excepté que la concentration de NaCl est ramenée à 135 mM) auquel on a ajouté 10 mM de chlorure de guanidinium plus 100-250 nCi de 14C-guanidinium, 10 µM de susbtance P, et un agoniste des récepteurs 5-HT₃ en présence ou en absence du composé à tester. L'incubation dure 10 min à 37°C. Le milieu est ensuite aspiré, et les cellules sont rapidement lavées deux fois avec 3 cm³ du tampon de lavage dans lequel le NaCl a été intégralement remplacé par une concentration équimolaire de chlorure de choline. Finalement, le tapis de cellules est repris dans 0,5 cm³ de soude 0,4 N, et la radioactivité accumulée est comptée par spectrométrie en milieu liquide (Aquasol).

Dans ces conditions expérimentales, les agonistes des récepteurs 5-HT₃ (10 µM - 0,1 mM) tels la sérotonine, la 2-méthyl-5-hydroxytryptamine, et le phénylbiguanide multiplient par 5 l'accumulation intracellulaire de 14C- guanidinium, et cet effet peut être complètement supprimé par 10-100 nM d'un antagoniste 5-HT₃ sélectif (ondansetron, zacopride).

Ce test a permis de montrer que certains composés de l'invention sont de puissants agonistes des récepteurs 5-HT₃.
En outre, d'autres composés de l'invention, agonistes s'ils sont testés seuls, se révèlent être antagonistes en présence de sérotonine.

Le tableau suivant illustre les effets des composés de l'invention :

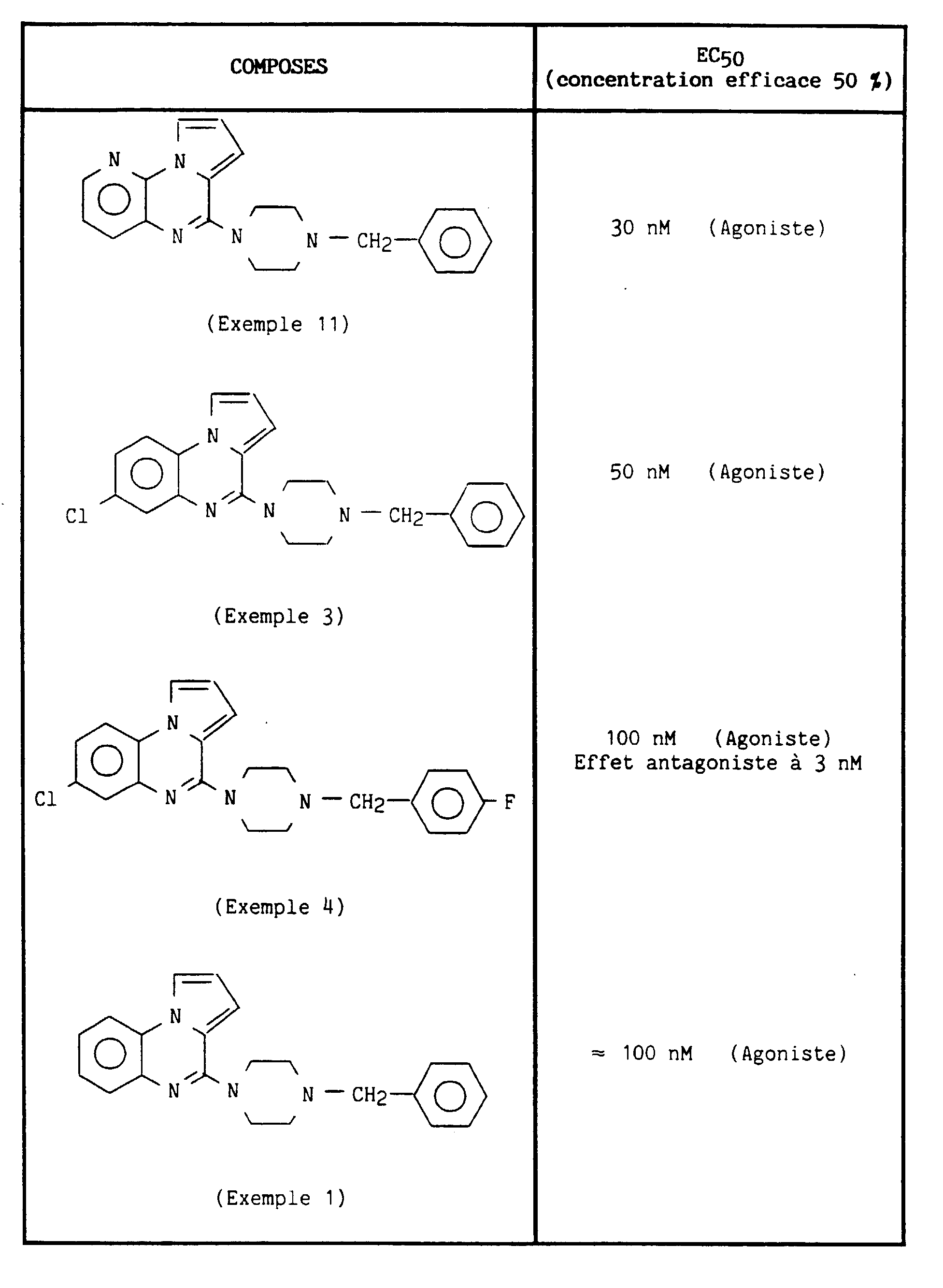

### EXEMPLE C : REFLEXE BRADYCARDIQUE DE BEZOLD-JARISCH

La bradycardie (réflexe de BEZOLD-JARISCH) est induite par l'injection intraveineuse de sérotonine 5-HT₃ chez le rat anesthésié à l'uréthane. Cette réponse consiste en une bradycardie brutale mais transitoire induite par la stimulation des récepteurs 5-HT₃ sur les afférences vagales. Ce modèle est donc tout à fait approprié pour révéler d'éventuels effets agonistes ou antagonistes de composés qui sont des ligands potentiels des récepteurs 5-HT₃.

### PROTOCOLE :

L'animal (rat mâle, adulte - souche Sprague-Dawley, 250-300 g) est tout d'abord anesthésié à l'uréthane (1,4 g/kg i.p.) puis trachéotomisé pour la mise en place d'une canule trachéale. Un cathéter (0,3 mm) est introduit dans l'aorte abdominale via l'artère fémorale pour l'enregistrement en continu de la pression artérielle et du rythme cardiaque. Les agents pharmacologiques sont injectés dans la veine saphène qui a été canulée à cet effet. L'injection i.v. de 30 µg de sérotonine, de 2-méthyl-5 hydroxytryptamine, ou de phénylbiguanide entraine une chute brutale (-80%) et transitoire (pendant environ 4-5 secondes) du rythme cardiaque, et cet effet peut être complètement empêché par l'administration (i.v.) préalable de divers antagonistes sélectifs tels le zacopride ou l'ondansetron (à la dose de 1 µg/kg).

### REFERENCE :

Drugs of the Future, 1992, 17(8) : 660-664.

### RESULTATS :

Il apparaît, dans ce test, que les composés de l'invention se comportent comme de puissants ligands des récepteurs 5-HT₃, et possèdent une action soit antagoniste, soit agoniste partiel.

Le tableau suivant illustre l'activité des composés de l'invention :

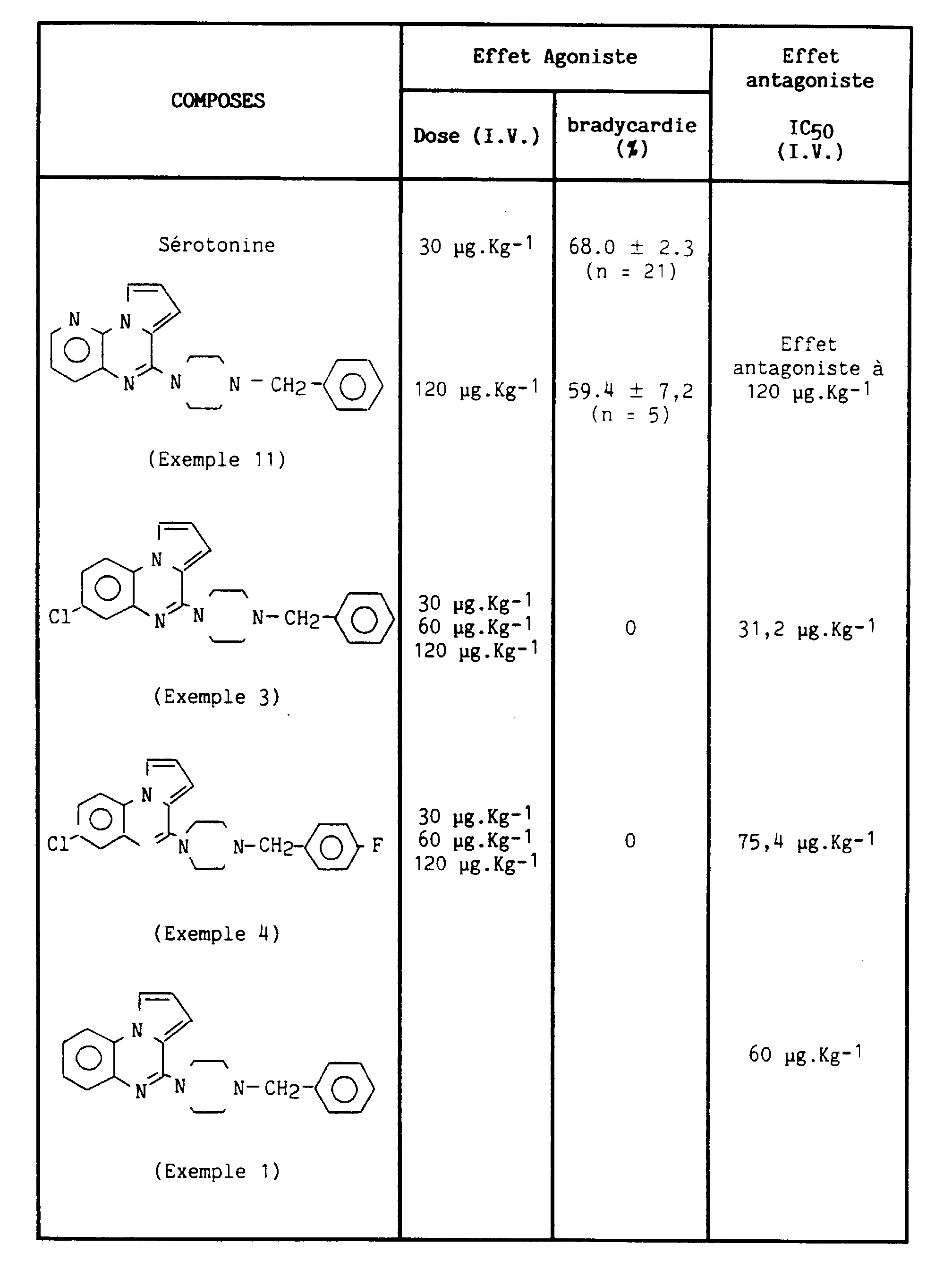

La bradycardie est exprimée en pourcentage de baisse (maximum) du rythme de base, avant tout traitement (moyennes ± E.S.M).

### EXEMPLE E : COMPOSITION PHARMACEUTIQUE : COMPRIMES

Comprimés dosés à 1 mg de 4-(4-benzylpipérazino)pyrrolo[1,2-a]quinoxaline

Formule de préparation pour 1000 comprimés :

| | |
|---|---|
| 4-(4-benzylpipérazino)pyrrolo[1,2-a]quinoxaline | 1 g |
| Amidon de blé | 2,5 g |
| Amidon de maïs | 1,5 g |
| Lactose | 8,5 g |
| Stéarate de magnésium | 0,2 g |
| Silice | 0,1 g |
| Hydroxypropylcellulose | 0,2 g |

## Revendications

1. Composés de formule (I) : 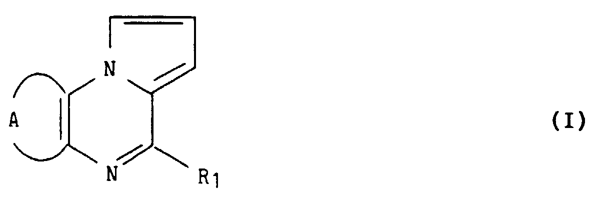 dans laquelle :
R₁ représente un groupement de formule : 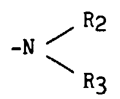 dans laquelle R₂ et R₃ forment avec l'atome d'azote qui les porte un groupement choisi parmi :
- pipérazine,
- pipérazine substituée,
- pipéridine,
- pipéridine substituée,
- pyrrolidine,
- pyrrolidine substituée,
- morpholine,
- morpholine substituée par un ou plusieurs radicaux alkyles,
- tétrahydropyridine,
- thiomorpholine,
- azaspirane de 5 à 12 chaînons,
- azaspirane de 5 à 12 chaînons substitué par un ou plusieurs radicaux alkyles ou groupements oxo,
- azacycloalkyle mono ou bicyclique de 7 à 12 chaînons incluant éventuellement dans son squelette de 1 à 2 hétéroatomes supplémentaires choisis parmi oxygène, soufre, et azote,
- azacycloalkyle mono ou bicyclique de 7 à 12 chaînons, substitue par un ou plusieurs radicaux alkyles ou groupements oxo, incluant éventuellement dans son squelette de 1 à 2 hétéroatomes supplémentaires choisis parmi oxygène, soufre, et azote,
- un groupement -NH-(CH₂)ₖ-NH₂ dans lequel k représente un nombre entier égal à 2, 3, ou 4,
- et un groupement -NH-(CH₂)ₖ-NH₂ substitué dans lequel k est tel que défini précédemment,
et A forme avec les 2 atomes de carbone auxquels il est lié un cycle choisi parmi benzo, pyrido, pyrazino, et pyrimidino ; A étant non substitué ou substitué par un ou plusieurs radicaux choisis parmi :
- alkyle,
- hydroxy,
- alkoxy,
- acyle,
- alkoxycarbonyle,
- halogène,
- trifluorométhyle,
- -(CH₂)ₘ-phényle et -O-(CH₂)ₘ-phényle dans lequel le noyau phényl est lui-même non substitué ou substitué par un ou plusieurs radicaux choisis parmi halogène, alkyle, alkoxy, hydroxy, et trifluorométhyle ; et m représente O ou un nombre entier de 1 à 4,
- -(CH₂)ₘ-pipérazine dans lequel le groupement pipérazine est lui-même substitué ou non substitué et m est tel que défini précédemment,
étant entendu que si A forme avec les 2 atomes de carbone auxquels il est lié un cycle benzo non substitué ou substitué par un trifluorométhyle ou un fluor, alors R₂ et R₃ ne peuvent pas former avec l'atome d'azote qui les porte une pipérazine non substituée ou substituée en position 4 par un radical méthyle ou (méthyl)phényle, et que si A forme avec les 2 atomes de carbone auxquels il est lié un cycle benzo non substitué, alors R₂ et R₃ ne peuvent pas former avec l'atome d'azote qui les porte une morpholine ou un groupement diméthylaminopropylamino,
étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux oxo, radicaux R₄, ou radicaux 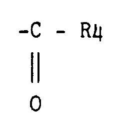 avec R₄ étant choisi parmi :
- alkyle,
- alkoxy,
- alcényle non substitué ou substitué par un radical phényle lui-même non substitué ou substitué par un ou plusieurs radicaux choisis parmi halogène, alkyle, alkoxy, hydroxy et trifluorométhyle,
- -(CH₂)ₙ-R₅ ou 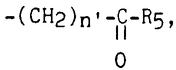 où n représente 0 ou un nombre entier de 1 à 5, n' représente un nombre entier de 1 à 5, et où R₅ représente un radical choisi parmi phényle, benzhydryle, thiényle, pyrrolyle, pyrrolidinyle, furyle, pyrimidinyle, pyridyle,méthylènedioxyphényle, éthylènedioxyphényle, naphtyle, quinoléinyle, isoquinoléinyle, cycloalkyle, et dicycloalkylméthyl ; le terme "cycloalkyle" représentant un groupement mono- ou bicyclique de 3 à 12 chaînons,
ces radicaux R₅ pouvant eux-mêmes être substitués par un ou plusieurs radicaux choisis parmi halogène, trifluorométhyle, carboxyle, hydroxyle, alkyle, et alkoxy,
- et -(CH₂)_{n'}-R₆ où n' est tel que défini précédemment et R₆ représente un groupement choisi parmi carboxyle, alkoxycarbonyle, amino, alkylamino, dialkylamino, -SO₂NR₇R₈, et -CONR₇R₈ dans lesquels R₇ et R₈ représentent chacun indépendamment l'un de l'autre un atome d'hydrogène ou un alkyle,
leurs isomères optiques,
et leurs sels d'addition à un acide ou à une base pharmaceutiquement acceptable,
étant entendu que, sauf précisions contraires,
- les termes "alkyle", "alkoxy", et "acyle" représentent des groupements linéaires ou ramifiés possédant de 1 à 6 atomes de carbone,
- et le terme "alcényle" représente un groupement insaturé, linéaire ou ramifié, possédant de 2 à 6 atomes de carbone.

2. Composés de formule (I) selon la revendication 1 dans lesquels A forme avec les 2 atomes de carbone auxquels il est lié un cycle benzo,
leurs isomères optiques,
et leurs sels d'addition à un acide ou à une base pharmaceutiquement acceptable.

3. Composés de formule (I) selon la revendication 1 dans lesquels A forme avec les 2 atomes de carbone auxquels il est lié un cycle pyrido,
leurs isomères optiques,
et leurs sels d'addition à un acide ou à une base pharmaceutiquement acceptable.

4. Composés de formule (I) selon la revendication 1 dans lesquels R₂ et R₃ forment avec l'atome d'azote qui les porte une pipérazine substituée en position 4 par un radical benzyle lui-même non substitué ou substitué,
leurs isomères optiques,
et leurs sels d'addition à un acide ou à une base pharmaceutiquement acceptable.

5. Composés selon la revendication 1 qui est la 4-(4-benzylpipérazino)-pyrrolo[1,2-a]quinoxaline et ses sels d'addition à un acide pharmaceutiquement acceptable.

6. Composé selon la revendication 1 qui est la 4-(4-benzylpipérazino)-7-chloro-pyrrolo[1,2-a]quinoxaline et ses sels d'addition à un acide pharmaceutiquement acceptable.

7. Composé selon la revendication 1 qui est la 7-chloro-4-[4-(3,4-méthylènedioxybenzyl)pipérazino]-pyrrolo[1,2-a]quinoxaline et ses sels d'addition à un acide pharmaceutiquement acceptable.

8. Composé selon la revendication 1 qui est la 6-(4-benzylpipérazino)-pyrido[3,2-e]pyrrolo[1,2-a]pyrazine et ses sels d'addition à un acide pharmaceutiquement acceptable.

9. Composé selon la revendication 1 qui est la 6-(4-allylpipérazino)-pyrido[3,2-e]pyrrolo[1,2-a]pyrazine et ses sels d'addition à un acide pharmaceutiquement acceptable.

10. Composé selon la revendication 1 qui est la 6-[4-(3,4-méthylènedioxybenzyl)piperazino]-pyrido[3,2-e]pyrrolo[1,2-a]pyrazine et ses sels d'addition à un acide pharmaceutiquement acceptable.

11. Composé selon la revendication 1 qui est la 6-(piperazino)-pyrido[3,2-e]pyrrolo[1,2-a]pyrazine et ses sels d'addition à un acide pharmaceutiquement acceptable.

12. Composé selon la revendication 1 qui est la 6-(4-methylpiperazino)-pyrido[3,2-e]pyrrolo[1,2-a]pyrazine et ses sels d'addition à un acide pharmaceutiquement acceptable.

13. Composé selon la revendication 1 qui est la 4-[4-(4-fluorobenzyl)piperazino]-7-methoxy-pyrrolo[1,2-a]quinoxaline et ses sels d'addition à un acide pharmaceutiquement acceptable.

14. Procédé de préparation des composés de formule (I) selon la revendication 1 caractérisé en ce que :
. soit on porte au reflux, en présence de phosgène, un composé de formule (II), 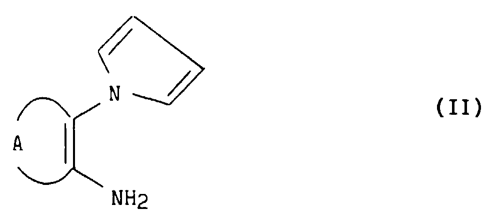 dans laquelle A est tel que défini dans la revendication 1,
. soit on chauffe, en présence ou en absence de solvant, un composé de formule (II/1), 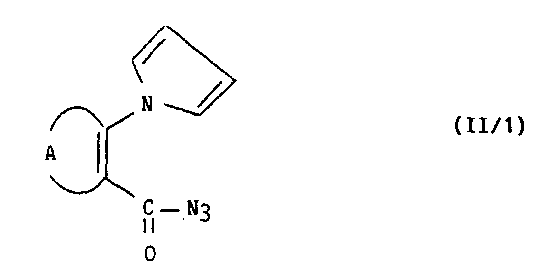 dans laquelle A est tel que défini dans la revendication 1,
pour obtenir un composé de formule (I/a) : 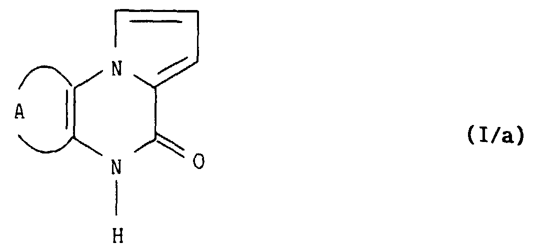 dans laquelle A est tel que défini précédemment,
composé de formule (I/a) qui est soumis à un agent d'halogénation pour obtenir un composé de formule (I/b) : 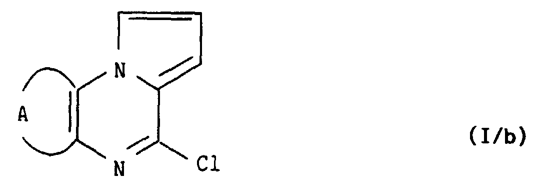 dans laquelle A est tel que défini précédemment,
qui est ensuite mis en réaction avec une amine de formule (III) : 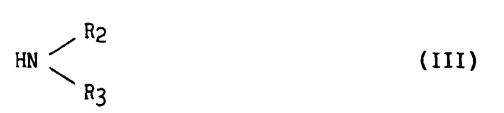 dans laquelle R₂ et R₃ sont tels que définis dans la revendication 1,
pour obtenir un composé de formule (I) selon la revendication 1 : 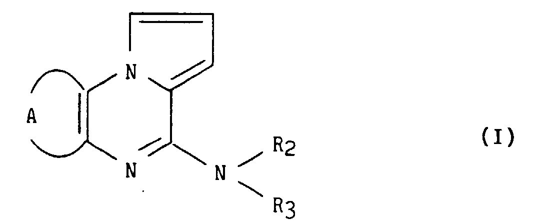 dans laquelle A, R₂, et R₃ sont tels que définis précédemment,
les composés de formule (I) selon la revendication 1 pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou par une base pharmaceutiquement acceptable.

15. Procédé de préparation des composés de formule (I), selon la revendication 1,
caractérisé en ce que :
on fait réagir un composé de formule (II') : 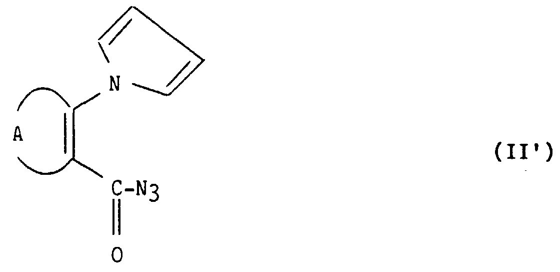 dans laquelle A est tel que défini dans la revendication 1,
avec une amine de formule (III) : 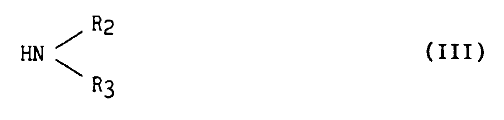 dans laquelle R₂ et R₃ sont tels que définis dans la revendication 1 pour obtenir un composé de formule (V) : 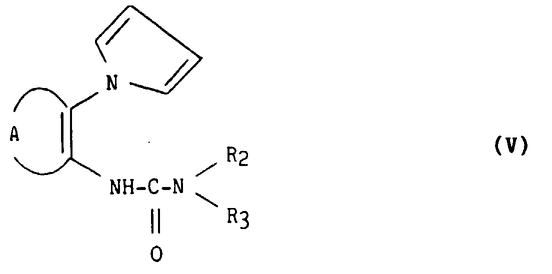 dans laquelle A, R₂ et R₃ sont tels que définis précédemment,
qui est ensuite cyclisé sous l'action d'un agent d'halogénation et soumis à un traitement alcalin pour donner un composé de formule (I) selon la revendication 1 : 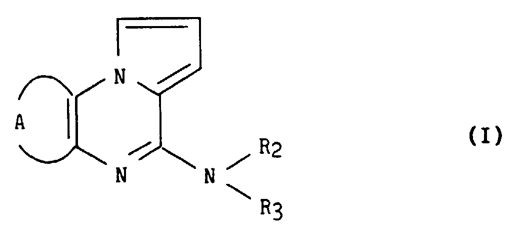 dans laquelle A, R₂, et R₃ sont tels que définis précédemment,
les composés de formule (I) selon la revendication 1 pouvant être :
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou par une base pharmaceutiquement acceptable.

16. Compositions pharmaceutiques renfermant comme principe actif au moins un des composés de formule (I) ou un de ses sels d'addition avec un acide ou avec une base pharmaceutiquement acceptable en combinaison avec un ou plusieurs excipients ou véhicules pharmaceutiquement acceptables.

17. Compositions pharmaceutiques selon la revendication 16 utiles dans la prévention et le traitement de l'anxiété, de la dépression, du stress, des psychoses, de la schizophrénie, des troubles du système nerveux central, de la migraine, des troubles de la mémoire, des troubles du comportement alimentaire, de l'alcoolisme, de la douleur, ainsi que dans la prévention et le traitement du vomissement et des désordres de la vidange gastrique.

## Patentansprüche

1. Verbindungen der Formel (I) 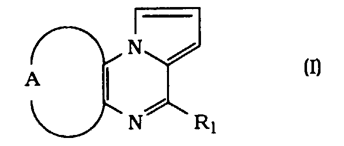 in der:
R₁ eine Gruppe der Formel: 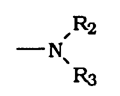 in der R₂ und R₃ zusammen mit dem sie tragenden Stickstoffatom eine Gruppe bilden ausgewählt aus:
- Piperazin.
- substituiertem Piperazin,
- Piperidin,
- substituiertem Piperidin,
- Pyrrolidin,
- substituiertem Pyrrolidin,
- Morpholin,
- durch eine oder mehrere Alkylgruppen substituiertem Morpholin,
- Tetrahydropyridin,
- Thiomorpholin,
- Azaspiran mit 5 bis 12 Kettengliedern,
- Azaspiran mit 5 bis 12 Kettengliedern, welches durch eine oder mehrere Alkylgruppen oder Oxogruppen substituiert ist,
- mono- oder bicyclischem Azacycloalkyl mit 7 bis 12 Kettengliedern, welches gegebenenfalls in seinem Gerüst 1 bis 2 zusätzliche Heteroatome ausgewählt aus Sauerstoff, Schwefel und Stickstoff aufweist,
- mono- oder bicyclischem Azacycloalkyl mit 7 bis 12 Kettengliedern, welches mit einer oder mehreren Alkylgruppen oder Oxogruppen substituiert ist und gegebenenfalls in seinem Gerüst 1 bis 2 zusätzliche Heteroatome ausgewählt aus Sauerstoff, Schwefel und Stickstoff enthält,
- einer Gruppe -NH-(CH₂)ₖ-NH₂, in der k eine ganze Zahl mit einem Wert von 2, 3 oder 4 darstellt, und
- einer substituierten Gruppe -NH-(CH₂)ₖ-NH₂, in der k die oben angegebenen Bedeutungen besitzt
und A zusammen mit den beiden Kohlenstoffatomen, an die es gebunden ist, einen Ring ausgewählt aus Benzo, Pyrido, Pyrazino und Pyrimidino bildet, wobei A unsubstituiert ist oder durch eine oder mehrere Gruppen ausgewählt aus:
- Alkyl,
- Hydroxyl,
- Alkoxy,
- Acyl,
- Alkoxycarbonyl,
- Halogen,
- Trifluormethyl,
- -(CH₂)ₘ-Phenyl und -O-(CH₂)ₘ-Phenyl, in der der Phenylkern seinerseits unsubstituiert oder durch eine oder mehrere Gruppen ausgewählt aus Halogen, Alkyl, Alkoxy, Hydroxy und Trifluormethyl substituiert ist, und m 0 oder eine ganze Zahl mit einem Wert von 1 bis 4 darstellt, und
- -(CH₂)ₘ-Piperazin, worin die Piperazingruppe ihrerseits substituiert oder unsubstituiert ist und m die oben angegebenen Bedeutungen besitzt, substituiert ist, bedeuten,
wobei es sich versteht, daß, wenn A zusammen mit den beiden Kohlenstoffatomen, an die es gebunden ist, einen Benzoring darstellt, der unsubstituiert oder durch eine Trifluormethylgruppe oder ein Fluoratom substituiert ist, R₂ und R₃ zusammen mit dem sie tragenden Stickstoffatom keinen Piperazinring bilden können, der unsubstituiert ist oder in der 4-Stellung durch eine Methylgruppe oder eine (Methyl)phenylgruppe substituiert ist, und wenn A zusammen mit den beiden Kohlenstoffatomen, an die es gebunden ist, einen unsubstituierten Benzoring bildet, R₂ und R₃ zusammen mit dem sie tragenden Stickstoffatom nicht eine Morpholingruppe oder eine Dimethylaminopropylaminogruppe bedeuten,
wobei es sich weiterhin versteht, daß der Begriff "substituiert", wie er die oben angesprochenen Piperazin-, Piperidin-, Pyrrolidin- und -NH-(HCH₂)ₖ-NH₂-Gruppen betrifft, bedeutet, daß diese Gruppen durch ein oder mehrere Halogenatome, Hydroxylgruppen, Oxogruppen, Gruppen R₄ oder Gruppen 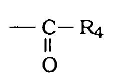 substituiert sein können, wobei R₄ ausgewählt ist aus:
- Alkyl,
- Alkoxy,
- Alkenyl, das unsubstituiert oder durch eine Phenylgruppe substituiert ist, die ihrerseits unsubstituiert oder durch eine oder mehrere Gruppen ausgewählt aus Halogen, Alkyl, Alkoxy, Hydroxyl und Trifluormethyl substituiert ist,
- -(CH₂)ₙ-R₅ oder 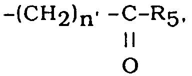 worin n 0 oder eine ganze Zahl mit einem Wert von 1 bis 5, n' eine ganze Zahl mit einem Wert von 1 bis 5 und R₅ eine Gruppe ausgewählt aus Phenyl, Benzhydryl, Thienyl, Pyrrolyl, Pyrrolidinyl, Furyl, Pyrimidinyl, Pyridyl, Methylendioxyphenyl, Ethylendioxyphenyl, Naphthyl, Chinolinyl, Isochinolinyl, Cycloalkyl und Dicycloalkylmethyl darstellen; wobei der Begriff "Cycloalkyl" eine mono- oder bicyclische Gruppe mit 3 bis 12 Kettengliedern darstellt,
wobei diese Gruppen R₅ ihrerseits unsubstituiert oder durch eine oder mehrere Gruppen ausgewählt aus Halogen, Trifluormethyl, Carboxyl, Hydroxyl, Alkyl und Alkoxy substituiert sein können,
- und -(CH₂)_{n'}-R₆, worin n' die oben angegebenen Bedeutungen besitzt und R₆ eine Gruppe ausgewählt aus Carboxyl, Alkoxycarbonyl, Amino, Alkylamino, Dialkylamino, -SO₂NR₇R₈ und -CONR₇R₈ darstellt, worin R₇ und R₈ jeweils unabhängig voneinander für ein Wasserstoffatom oder eine Alkylgruppe stehen, bedeuten,
deren optische Isomere,
und deren Additionssalze mit einer pharmazeutisch annehmbaren Säure oder Base, wobei es sich versteht, daß, wenn nichts anderes angegeben ist,
- die Begriffe "Alkyl", "Alkoxy" und "Acyl" für geradkettige oder verzweigte Gruppen mit 1 bis 6 Kohlenstoffatomen stehen und
- der Begriff "Alkenyl" für eine ungesättigte, geradkettige oder verzweigte Gruppe mit 2 bis 6 Kohlenstoffatomen steht.

2. Verbindungen der Formel (I) nach Anspruch 1, in der A mit den 2 Kohlenstoffatomen, an die es gebunden ist, einen Benzoring bildet,
deren optische Isomere
und deren Additionssalze mit einer pharmazeutisch annehmbaren Säure oder Base.

3. Verbindungen der Formel (I) nach Anspruch 1, in der A zusammen mit den beiden Kohlenstoffatomen, an die er gebunden ist, einen Pyridoring bildet,
deren optische Isomere
und deren Additionssalze mit einer pharmazeutisch annehmbaren Säure oder Base.

4. Verbindungen der Formel (I) nach Anspruch 1, in der R₂ und R₃ zusammen mit dem sie tragenden Stickstoffatom einen Piperazinrest bilden, der in der 4-Stellung durch einen Benzylrest substituiert ist, der seinerseits unsubstituiert oder substituiert ist,
deren optische Isomere
und deren Additionssalze mit einer pharmazeutisch annehmbaren Säure oder Base.

5. Verbindung nach Anspruch 1, nämlich 4-(4-Benzylpiperazino)-pyrrolo[1,2-a]chinoxalin und dessen Additionssalze mit einer pharmazeutisch annehmbaren Säure.

6. Verbindung nach Anspruch 1, nämlich 4-(4-Benzylpiperazino)-7-chlor-pyrrolo[1,2-a]chinoxalin und dessen Additionssalze mit einer pharmazeutisch annehmbaren Säure.

7. Verbindung nach Anspruch 1, nämlich 7-Chlor-4-[4-(3,4-methylendioxybenzyl)-perazino]-pyrrolo[1,2-a]chinoxalin und dessen Additionssalze mit einer pharmazeutisch annehmbaren Säure.

8. Verbindung nach Anspruch 1, nämlich 6-(4-Benzylpiperazinol-pyrido[3,2-e]pyrrolo[1,2-a]pyrazin und dessen Additionssalze mit einer pharmazeutisch annehmbaren Säure.

9. Verbindung nach Anspruch 1, nämlich 6-(4-Allylpiperazino) -pyrido[3,2-e]pyrrolo[1,2-a]pyrazinund dessen Additionssalze mit einer pharmazeutisch annehmbaren Säure.

10. Verbindung nach Anspruch 1, nämlich 6-[4-[3,4-Methylendioxybenzyl)-piperazinol-pyrido[3,2-e]pyrrolo[1,2-a]pyrazin und dessen Additionssalze mit einer pharmazeutisch annehmbaren Säure.

11. Verbindung nach Anspruch 1, nämlich 6-(Piperazino)-pyrido[3,2-e]pyrrolo[1,2-a]pyrazin und dessen Additionssalze mit einer pharmazeutisch annehmbaren Säure.

12. Verbindung nach Anspruch 1. nämlich 6-(4-Methylpiperazino)-pyrido[3,2-e]pyrrolo[1,2-a]pyrazin und dessen Additionssalze mit einer pharmazeutisch annehmbaren Säure.

13. Verbindung nach Anspruch 1, nämlich 4-[4-(4-Fluorbenzyl)-piperazino]-7-methoxy-pyrrolo[1,2-a]chinoxalin und dessen Additionssalze mit einer pharmazeutisch annehmbaren Säure.

14. Verfahren zur Herstellung der Verbindungen der Formel (I) nach Anspruch 1, dadurch gekennzeichnet, daß man:
· entweder eine Verbindung der Formel (II) 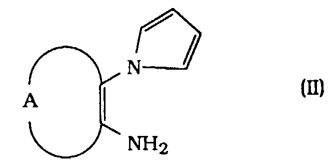 in der A die in Anspruch 1 angegebenen Bedeutungen besitzt, in Gegenwart von Phosgen zum Sieden am Rückfluß erhitzt,
· oder eine Verbindung der Formel (II/1) 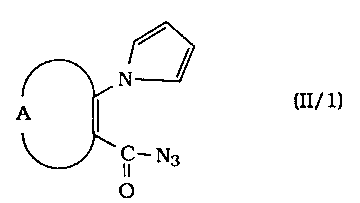 in der A die in Anspruch 1 angegebenen Bedeutungen besitzt,
in Gegenwart oder in Abwesenheit eines Lösungsmittels erhitzt zur Bildung einer Verbindung der Formel (I/a): 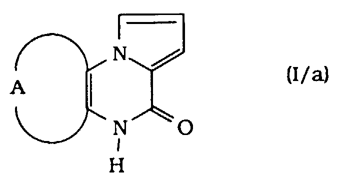 in der A die oben angegebenen Bedeutungen besitzt,
welche Verbindung der Formel (I/a) der Einwirkung eines Halogenierungsmittels unterworfen wird zur Bildung einer Verbindung der Formel (I/b): 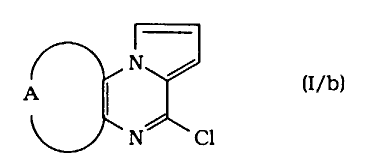 in der A die oben angegebenen Bedeutungen besitzt,
die anschließend mit einem Amin der Formel (III): 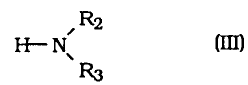 in der R₂ und R₃ die in Anspruch 1 angegebenen Bedeutungen besitzen, umgesetzt wird zur Bildung einer Verbindung der Formel (I) nach Anspruch 1: 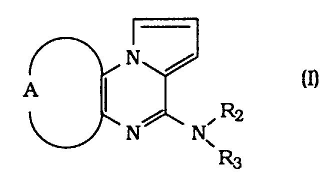 in der A, R₂ und R₃ die oben angegebenen Bedeutungen besitzen,
wobei die Verbindungen der Formel (I) nach Anspruch 1:
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung getrennt werden können
- und/oder mit einer pharmazeutisch annehmbaren Säure oder Base in die Salze überführt werden können.

15. Verfahren zur Herstellung der Verbindungen der Formel (I) nach Anspruch 1, dadurch gekennzeichnet, daß man:
eine Verbindung der Formel (II'): 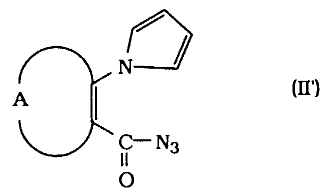 in der A die in Anspruch 1 angegebenen Bedeutungen besitzt,
mit einem Amin der Formel (III): 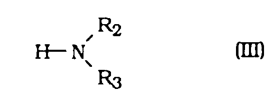 in der R₂ und R₃ die in Anspruch 1 angegebenen Bedeutungen besitzen, umsetzt zur Bildung einer Verbindung der Formel (V): 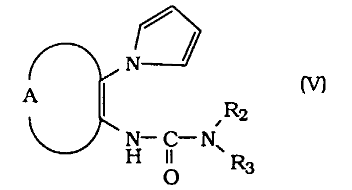 in der A, R₂ und R₃ die oben angegebenen Bedeutungen besitzen,
welche anschließend durch Einwirkung eines Halogenierungsmittels und Behandeln mit einem alkalischen Mittel cyclisiert wird zur Bildung einer Verbindung der Formel (I) nach Anspruch 1: 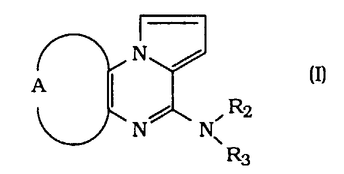 in der A, R₂ und R₃ die oben angegebenen Bedeutungen besitzen,
wobei die Verbindungen der Formel (I) nach Anspruch 1:
- mit Hilfe einer oder mehrerer Reinigungsmethoden ausgewählt aus Kristallisation, Säulenchromatographie über Siliciumdioxid, Extraktion, Filtration und Überführung über Kohlenstoff und/oder ein Harz gereinigt werden können,
- gegebenenfalls in ihre eventuellen optischen Isomeren in reiner Form oder in Form einer Mischung getrennt werden können
- und/oder mit Hilfe einer pharmazeutisch annehmbaren Säure oder Base in ihre Salze überführt werden können.

16. Pharmazeutische Zubereitungen enthaltend als Wirkstoff mindestens eine Verbindung der Formel (I) oder eines ihrer Additionssalze mit einer pharmazeutisch annehmbaren Säure oder Base in Kombination mit einem oder mehreren pharmazeutisch annehmbaren Trägermaterialien oder Bindemitteln.

17. Pharmazeutische Zubereitung nach Anspruch 16 zur Vorbeugung und zur Behandlung der Angst, der Depression, von Streß, Psychosen, der Schizophrenie, von Störungen des Zentralnervensystems, der Migräne, von Gedächtnisstörungen, von Ernährungsverhaltensstörungen, des Alkoholismus, von Schmerzen sowie zur Vorbeugung und zur Behandlung des Erbrechens und von Störungen der Magenentleerung.

## Claims

1. Compounds of formula (I): 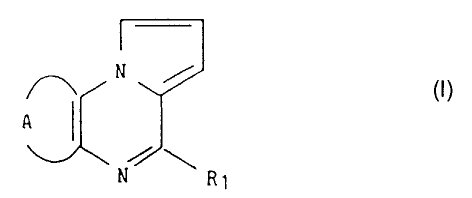 wherein:
R₁ represents a group of formula : 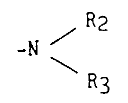 wherein R₂ and R₃ form, with the nitrogen atom that carries them, a group selected from :
- piperazine,
- substituted piperazine,
- piperidine,
- substituted piperidine,
- pyrrolidine,
- substituted pyrrolidine,
- morpholine,
- morpholine substituted by one or more alkyl radicals,
- tetrahydropyridine,
- thiomorpholine,
- azaspirane having from 5 to 12 ring members,
- azaspirane having from 5 to 12 ring members, substituted by one or more alkyl radicals or oxo groups,
- mono- or bi-cyclic azacycloalkyl having from 7 to 12 ring members, optionally including in its skeleton 1 or 2 additional hetero atoms selected from oxygen, sulphur and nitrogen,
- mono- or bi-cyclic azacycloalkyl having from 7 to 12 ring members, substituted by one or more alkyl radicals or oxo groups, optionally including in its skeleton 1 or 2 additional hetero atoms selected from oxygen, sulphur and nitrogen,
- a -NH-(CH₂)ₖ-NH₂ group wherein k represents an integer equal to 2, 3 or 4, and
- a substituted -NH-(CH₂)ₖ-NH₂ group wherein k is as defined hereinabove,
and A forms, with the 2 carbon atoms to which it is bonded, a ring selected from benzo, pyrido, pyrazino and pyrimidino; A being unsubstituted or substituted by one or more radicals selected from:
- alkyl,
- hydroxy,
- alkoxy,
- acyl,
- alkoxycarbonyl,
- halogen,
- trifluoromethyl,
- -(CH₂)ₘ-phenyl and -O-(CH₂)ₘ-phenyl wherein the phenyl nucleus is itself unsubstituted or substituted by one or more radicals selected from halogen, alkyl, alkoxy, hydroxy and trifluoromethyl; and m represents 0 or an integer from 1 to 4,
- -(CH₂)ₘ-Piperazine wherein the piperazine group is itself substituted or unsubstituted and m is as defined hereinabove,
it being understood that if A forms, with the 2 carbon atoms to which it is bonded, a benzo ring that is unsubstituted or substituted by trifluoromethyl or fluorine, then R₂ and R₃ cannot form, with the nitrogen atom that carries them, a piperazine that is unsubstituted or substituted in the 4-position by a methyl or methylphenyl radical, and if A forms, with the 2 carbon atoms to which it is bonded, an unsubstituted benzo ring, then R₂ and R₃ cannot form, with the nitrogen atom that carries them, morpholine or a dimethylaminopropylamino group,
it being understood that the term "substituted" used above in respect of the piperazine, piperidine, pyrrolidine and -NH-(CH₂)ₖ-NH₂ groups means that those groups may be substituted by one or more halogen atoms, hydroxy radicals, oxo radicals, R₄ radicals or 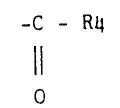 radicals
R₄ being selected from:
- alkyl,
- alkoxy,
- alkenyl unsubstituted or substituted by a phenyl radical itself unsubstituted or substituted by one or more radicals selected from halogen, alkyl, alkoxy, hydroxy and trifluoromethyl,
- -(CH₂)ₙ-R₅ and 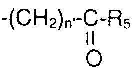 wherein n represents 0 or an integer from 1 to 5, n' represents an integer from 1 to 5 and wherein R₅ represents a radical selected from phenyl, benzhydryl, thienyl, pyrrolyl, pyrrolidinyl, furyl, pyrimidinyl, pyridyl, methylenedioxyphenyl, ethylenedioxyphenyl, naphthyl, quinolyl, isoquinolyl, cycloalkyl and dicycloalkylmethyl; the term "cycloalkyl" representing a mono- or bi-cyclic group having from 3 to 12 ring members,
it being possible for those R₅ radicals themselves to be substituted by one or more radicals selected from halogen, trifluoromethyl, carboxy, hydroxy, alkyl and alkoxy,
- and -(CH₂)_{n'}-R₆ wherein n' is as defined hereinabove and R₆ represents a group selected from carboxy, alkoxycarbonyl, amino, alkylamino, dialkylamino, -SO₂NR₇R₈ and -CONR₇R₈ wherein R₇ and R₈ each independently of the other represent a hydrogen atom or alkyl,
their optical isomers,
and addition salts thereof with a pharmaceutically acceptable acid or base,
it being understood that, unless indicated to the contrary,
- the terms "alkyl", "alkoxy" and "acyl" represent linear or branched groups having from 1 to 6 carbon atoms, and
- the term "alkenyl" represents an unsaturated linear or branched group having from 2 to 6 carbon atoms.

2. Compounds of formula (I) according to claim 1 wherein A forms, with the 2 carbon atoms to which it is bonded, a benzo ring,
their optical isomers,
and addition salts thereof with a pharmaceutically acceptable acid or base.

3. Compounds of formula (I) according to claim 1 wherein A forms, with the 2 carbon atoms to which it is bonded, a pyrido ring,
their optical isomers,
and addition salts thereof with a pharmaceutically acceptable acid or base.

4. Compounds of formula (I) according to claim 1 wherein R₂ and R₃ form, with the nitrogen atom that carries them, a piperazine substituted in the 4-position by a benzyl radical which is itself unsubstituted or substituted,
their optical isomers,
and addition salts thereof with a pharmaceutically acceptable acid or base.

5. Compound according to claim 1 which is 4-(4-benzylpiperazino)-pyrrolo[1,2-a]quinoxaline and addition salts thereof with a pharmaceutically acceptable acid.

6. Compound according to claim 1 which is 4-(4-benzylpiperazino)-7-chloropyrrolo[1,2-a]quinoxaline and addition salts thereof with a pharmaceutically acceptable acid.

7. Compound according to claim 1 which is 7-chloro-4-[4-(3,4-methylenedioxybenzyl)piperazino]-pyrrolo[1,2-a]quinoxaline and addition salts thereof with a pharmaceutically acceptable acid.

8. Compound according to claim 1 which is 6-(4-benzylpiperazino)-pyrido[3,2-e]pyrrolo[1,2-a]pyrazine and addition salts thereof with a pharmaceutically acceptable acid.

9. Compound according to claim 1 which is 6-(4-allylpiperazino)-pyrido[3,2-e]pyrrolo[1,2-a]pyrazine and addition salts thereof with a pharmaceutically acceptable acid.

10. Compound according to claim 1 which is 6-[4-(3,4-methylenedioxybenzyl)-piperazino]-pyrido[3,2-e]pyrrolo[1,2-a]pyrazine and addition salts thereof with a pharmaceutically acceptable acid.

11. Compound according to claim 1 which is 6-(piperazino)-pyrido[3,2-e]pyrrolo[1,2-a]pyrazine and addition salts thereof with a pharmaceutically acceptable acid.

12. Compound according to claim 1 which is 6-(4-methylpiperazino)-pyrido[3,2-e]pyrrolo[1,2-a]pyrazine and addition salts thereof with a pharmaceutically acceptable acid.

13. Compound according to claim 1 which is 4-[4-(4-fluorobenzyl)piperazino]-7-methoxy-pyrrolo[1,2-a]quinoxaline and addition salts thereof with a pharmaceutically acceptable acid.

14. Process for the preparation of compounds of formula (I) according to claim 1, characterised in that either:
• a compound of formula (II) 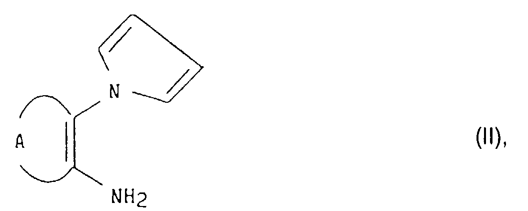 wherein A is as defined in claim 1, is refluxed, in the presence of phosgene, or
• a compound of formula (II/1) 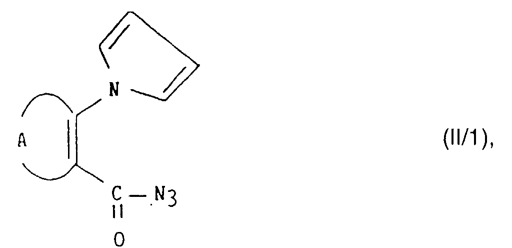 wherein A is as defined in claim 1, is heated, in the presence or absence of a solvent,
to obtain a compound of formula (I/a): 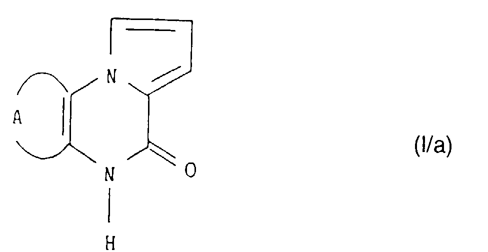 wherein A is as defined hereinabove
which compound of formula (I/a) is subjected to a halogenation agent to obtain a compound of formula (I/b) : 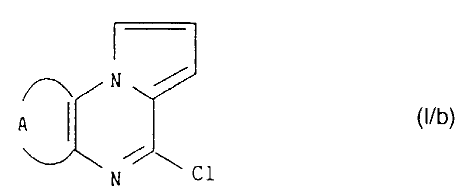 wherein A is as defined hereinabove,
which is then reacted with an amine of formula (III) : 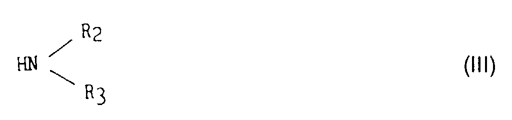 wherein R₂ and R₃ are as defined in claim 1,
to obtain a compound of formula (I) according to claim 1 : 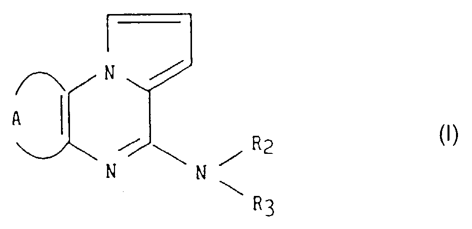 wherein A, R₂ and R₃ are as defined hereinabove,
which compounds of formula (I) according to claim 1 can be :
- purified in accordance with one or more purification methods selected from crystallisation, chromatography on a silica column, extraction, filtration and passage over charcoal and/or resin,
- separated, where appropriate, in pure form or in the form of a mixture, into their possible optical isomers, and/or
- converted into salts by a pharmaceutically acceptable acid or base.

15. Process for the preparation of compounds of formula (I) according to claim 1, characterised in that :
a compound of formula (II') : 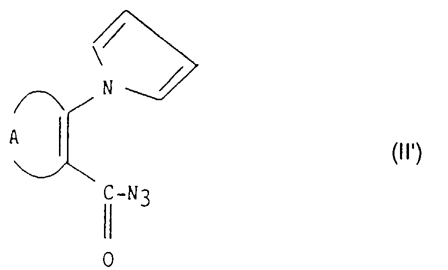 wherein A is as defined in claim 1,
is reacted with an amine of formula (III) : 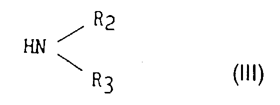 wherein R₂ and R₃ are as defined in claim 1
to obtain a compound of formula (V) : 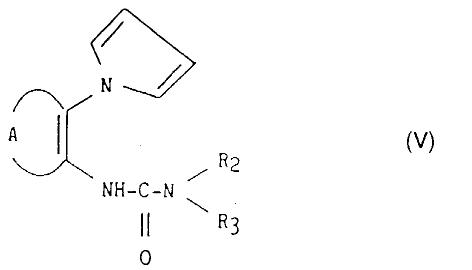 wherein A, R₂ and R₃ are as defined hereinabove,
which is then cyclised under the action of a halogenation agent and subjected to alkaline treatment to yield a compound of formula (I) according to claim 1 : 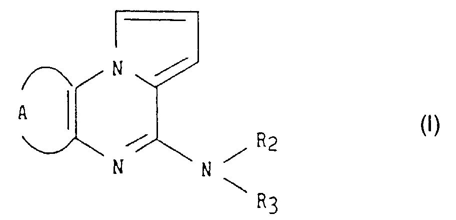 wherein A, R₂ and R₃ are as defined hereinabove,
which compounds of formula (I) according to claim 1 can be :
- purified in accordance with one or more purification methods selected from crystallisation, chromatography on a silica column, extraction, filtration and passage over charcoal and/or resin,
- separated, where appropriate, in pure form or in the form of a mixture, into their possible optical isomers, and/or
- converted into salts by a pharmaceutically acceptable acid or base.

16. Pharmaceutical compositions comprising as active ingredient at least one of the compounds of formula (I) or an addition salt thereof with a pharmaceutically acceptable acid or base, in combination with one or more pharmaceutically acceptable excipients or carriers.

17. Pharmaceutical compositions according to claim 16 for use in the prevention and treatment of anxiety, depression, stress, psychoses, schizophrenia, disorders of the central nervous system, migraine, memory disorders, eating disorders, alcoholism and pain, and in the prevention and treatment of vomiting and gastric emptying disorders.
